(19) Europäisches Patentamt / European Patent Office / Office européen des brevets

(11) EP 3 219 798 B1

(12) **EUROPEAN PATENT SPECIFICATION**

(45) Date of publication and mention of the grant of the patent:
**17.07.2019 Bulletin 2019/29**

(21) Application number: **15858310.4**

(22) Date of filing: **10.11.2015**

(51) Int Cl.:
*C12N 15/09* (2006.01) *C12N 15/33* (2006.01) *C12N 15/10* (2006.01) *A61K 31/7088* (2006.01) *A61P 25/04* (2006.01)

(86) International application number:
**PCT/RU2015/000756**

(87) International publication number:
**WO 2016/076761 (19.05.2016 Gazette 2016/20)**

(54) **DNA PLASMID, CODING HNP-1, OR HNP-2, OR HNP-3, BACTERIAL PRODUCER, ANALGESIC AGENT (VARIANTS)**

FÜR HNP-1 ODER HNP-2 ODER HNP-3 CODIERENDES DNS-PLASMID, BAKTERIELLER ERZEUGER, ANALGETIKUM (VARIANTEN)

ADN PLASMIDIQUE CODANT POUR HNP-1 OU HNP-2 OU HNP-3, PRODUCTEUR BACTÉRIEN, PRODUIT ANALGÉSIQUE (ET VARIANTES)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.11.2014 RU 2014145169**

(43) Date of publication of application:
**20.09.2017 Bulletin 2017/38**

(73) Proprietors:
- **Dukhovlinov, Ilya Vladimirovich**
  **Saint-Petersburg, 190068 (RU)**
- **Orlov, Anton Iosifovich**
  **Saint-Petersburg 197350 (RU)**

(72) Inventors:
- **Dukhovlinov, Ilya Vladimirovich**
  **Saint-Petersburg, 190068 (RU)**
- **Orlov, Anton Iosifovich**
  **Saint-Petersburg 197350 (RU)**

(74) Representative: **Lapienis, Juozas**
**MSP Europe UAB**
**21-92 Seimyniskiu Str.**
**09236 Vilnius (LT)**

(56) References cited:

| | |
|---|---|
| **WO-A1-02/064832** | **WO-A1-2007/057617** |
| **WO-A1-2009/043461** | **WO-A2-2006/097110** |
| **WO-A2-2013/151668** | **CN-A- 101 648 011** |
| **US-A1- 2002 182 703** | **US-A1- 2006 036 083** |

- **DATABASE Geneseq [Online] 15 June 2007 (2007-06-15), "Human defensin 1.", XP002778943, retrieved from EBI accession no. GSP:ABB98494 Database accession no. ABB98494**
- **DATABASE JPO Proteins [Online] 24 August 2012 (2012-08-24), "JP 2011516026-A/4177: COMPOSITIONS AND METHODS FOR THE TREATMENT OF IMMUNE RELATED DISEASES.", XP002778944, retrieved from EBI accession no. JPOP:DJ776437 Database accession no. DJ776437**
- **JAMES A WILLIAMS ET AL.: 'Plasmid DNA Vaccine vector design: impact on efficacy, safety and upstream production' BIOTECHNOL ADV. vol. 27, no. 4, 01 July 2009, pages 353 - 370, XP002594537 DOI: 10.1016/J.BIOTECHADV.2009.02.003**
- **DAVID M.HOOVER ET AL.: 'DNAWorks: an automated method for designing oligonucleotides for PCR-based gene synthesis' NUCLEIC ACIDS RESEARCH vol. 30, no. 10, 15 May 2002, pages 1 - 7, XP002301503 DOI: 10.1093/NAR/30.10.E43**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

Sequence Listing

<110> Dukhovlinov I.v., Orlov A.I.
<120> Plasmid DNA encoding HNP-1 or HNP-2 or HNP-3, bacterial producer, analgesic agent (emb.)
<130>
<150> RU 2014145169
<151> 2014-11-10

<160> 12
<210> 1
<211> 94
<212> PRT
<213> Homo sapiens
<220>
<223> neutrophil defensin 1 preproprotein
<400> 1

```
Met Arg Thr Leu Ala Ile Leu Ala Ala Ile Leu Leu Val Ala Leu Gln
  1               5                  10                  15
Ala Gln Ala Glu Pro Leu Gln Ala Arg Ala Asp Glu Val Ala Ala Ala
            20                  25                  30
Pro Glu Gln Ile Ala Ala Asp Ile Pro Glu Val Val Val Ser Leu Ala
        35                  40                  45
Trp Asp Glu Ser Leu Ala Pro Lys His Pro Gly Ser Arg Lys Asn Met
    50                  55                  60
Ala Cys Tyr Cys Arg Ile Pro Ala Cys Ile Ala Gly Glu Arg Arg Tyr
65                  70                  75                  80
Gly Thr Cys Ile Tyr Gln Gly Arg Leu Trp Ala Phe Cys Cys
                85                  90
```

<210> 2
<211> 94
<212> PRT
<213> Homo sapiens
<220>
<223> neutrophil defensin 3 preproprotein
<400> 2

```
Met Arg Thr Leu Ala Ile Leu Ala Ala Ile Leu Leu Val Ala Leu Gln
  1               5                  10                  15
Ala Gln Ala Glu Pro Leu Gln Ala Arg Ala Asp Glu Val Ala Ala Ala
            20                  25                  30
Pro Glu Gln Ile Ala Ala Asp Ile Pro Glu Val Val Val Ser Leu Ala
        35                  40                  45
Trp Asp Glu Ser Leu Ala Pro Lys His Pro Gly Ser Arg Lys Asn Met
    50                  55                  60
Asp Cys Tyr Cys Arg Ile Pro Ala Cys Ile Ala Gly Glu Arg Arg Tyr
65                  70                  75                  80
Gly Thr Cys Ile Tyr Gln Gly Arg Leu Trp Ala Phe Cys Cys
                85                  90
```

<210> 3
<211> 417
<212> DNA
<213> Artificial sequence
<220>
<223> DNA coding for neutr. def. 1 preproprot. [Homo sapiens] cod.-opt. for
expr. in mamm., nat. term.
seq.
<400> 3

```
atgagaaccc tggccatcct ggccgccatc ctgctggtgg ccctgcaggc ccaggccgag  60
cccctgcagg ccagagccga cgaggtggcc gccgcccccg agcagatcgc cgccgacatc 120
```

```
cccgaggtgg tggtgagcct ggcctgggac gagagcctgg cccccaagca ccccggcagc 180
agaaagaaca tggcctgcta ctgcagaatc cccgcctgca tcgccggcga gagaagatac 240
ggcacctgca tctaccaggg cagactgtgg gccttctgct gctgagcttg cagaaaaaga 300
aaaatgagct caaaatttgc tttgagagct acagggaatt gctattactc ctgtaccttc 360
tgctcaattt cctttcctca tcccaaataa atgccttgaa aaaaaaaaaa aaaatga 417
```

<210> 4
<211> 417
<212> DNA
<213> Artificial sequence
<220>
<223> DNA coding for neutr. def. 3 preproprot. [Homo sapiens] cod.-opt. for expr. in mamm., nat. term. seq.
<400> 4

```
ATGAGAACCC TGGCCATCCT GGCCGCCATC CTGCTGGTGG CCCTGCAGGC CCAGGCCGAG  60
CCCCTGCAGG CCAGAGCCGA CGAGGTGGCC GCCGCCCCCG AGCAGATCGC CGCCGACATC 120
CCCGAGGTGG TGGTGAGCCT GGCCTGGGAC GAGAGCCTGG CCCCCAAGCA CCCCGGCAGC 180
AGAAAGAACA TGGACTGCTA CTGCAGAATC CCCGCCTGCA TCGCCGGCGA GAGAAGATAC 240
GGCACCTGCA TCTACCAGGG CAGACTGTGG GCCTTCTGCT GCTGAGCTTG CAGAAAAAGA 300
AAAATGAGCT CAAAATTTGC TTTGAGAGCT ACAGGGAATT GCTATTACTC CTGTACCTTC 360
TGCTCAATTT CCTTTCCTCA TCTCAAATAA ATGCCTTGTT ACAAAAAAAA AAAATGA 417
```

<210> 5
<211> 288
<212> DNA
<213> Artificial sequence
<220>
<223> DNA coding for neutr. def. 1 preproprot. [Homo sapiens] cod.-opt. for expr. in mamm., stop-cod.
<400> 5

```
atgagaaccc tggccatcct ggccgccatc ctgctggtgg ccctgcaggc ccaggccgag  60
cccctgcagg ccagagccga cgaggtggcc gccgcccccg agcagatcgc cgccgacatc 120
cccgaggtgg tggtgagcct ggcctgggac gagagcctgg cccccaagca ccccggcagc 180
agaaagaaca tggcctgcta ctgcagaatc cccgcctgca tcgccggcga gagaagatac 240
ggcacctgca tctaccaggg cagactgtgg gccttctgct gctgataa 288
```

<210> 6
<211> 288
<212> DNA
<213> Artificial sequence
<220>
<223> DNA coding for neutr. def. 3 preproprot. [Homo sapiens] cod.-opt. for expr. in mamm., stop-cod.
<400> 6

```
atgagaaccc tggccatcct ggccgccatc ctgctggtgg ccctgcaggc ccaggccgag  60
cccctgcagg ccagagccga cgaggtggcc gccgcccccg agcagatcgc cgccgacatc 120
cccgaggtgg tggtgagcct ggcctgggac gagagcctgg cccccaagca ccccggcagc 180
agaaagaaca tggactgcta ctgcagaatc cccgcctgca tcgccggcga gagaagatac 240
ggcacctgca tctaccaggg cagactgtgg gccttctgct gctgataa 288
```

<210> 7
<211> 98
<212> PRT
<213> Artificial sequence

<220>
<223> neutrophil defensin 1 proprotein [Homo sapiens] with TPA signal sequence
<400> 7

```
Met Asp Ala Met Lys Arg Gly Leu Cys Cys Val Leu Leu Leu Cys Gly
  1             5                  10                  15
Ala Val Phe Val Ser Pro Ser Glu Pro Leu Gln Ala Arg Ala Asp Glu
            20                  25                  30
Val Ala Ala Ala Pro Glu Gln Ile Ala Ala Asp Ile Pro Glu Val Val
        35                  40                  45

Val Ser Leu Ala Trp Asp Glu Ser Leu Ala Pro Lys His Pro Gly Ser
    50                  55                  60
Arg Lys Asn Met Ala Cys Tyr Cys Arg Ile Pro Ala Cys Ile Ala Gly
65                  70                  75                  80
Glu Arg Arg Tyr Gly Thr Cys Ile Tyr Gln Gly Arg Leu Trp Ala Phe
                85                  90                  95
Cys Cys
```

<210> 8
<211> 98
<212> PRT
<213> Artificial sequence
<220>
<223> neutrophil defensin 3 proprotein [Homo sapiens] with TPA signal sequence
<400> 8

```
Met Asp Ala Met Lys Arg Gly Leu Cys Cys Val Leu Leu Leu Cys Gly
  1             5                  10                  15
Ala Val Phe Val Ser Pro Ser Glu Pro Leu Gln Ala Arg Ala Asp Glu
            20                  25                  30
Val Ala Ala Ala Pro Glu Gln Ile Ala Ala Asp Ile Pro Glu Val Val
        35                  40                  45
Val Ser Leu Ala Trp Asp Glu Ser Leu Ala Pro Lys His Pro Gly Ser
    50                  55                  60
Arg Lys Asn Met Asp Cys Tyr Cys Arg Ile Pro Ala Cys Ile Ala Gly
65                  70                  75                  80
Glu Arg Arg Tyr Gly Thr Cys Ile Tyr Gln Gly Arg Leu Trp Ala Phe
                85                  90                  95
Cys Cys
```

<210> 9
<211> 429
<212> DNA
<213> Artificial sequence
<220>
<223> DNA enc.neutr.def.1 proprot.[H.sap.]with TPA sig.seq.,cod.opt.for expr.in mam.,nat.term.seq.
<400> 9

```
atggacgcca tgaagagagg cctgtgctgc gtgctgctgc tgtgcggcgc cgtgttcgtg  60
agccccagcg agcccctgca ggccagagcc gacgaggtgg ccgccgcccc cgagcagatc 120
gccgccgaca tccccgaggt ggtggtgagc ctggcctggg acgagagcct ggcccccaag 180
cacccccggca gcagaaagaa catggcctgc tactgcagaa tccccgcctg catcgccggc 240
gagagaagat acggcacctg catctaccag ggcagactgt gggccttctg ctgctgagct 300
tgcagaaaaa gaaaaatgag ctcaaaattt gctttgagag ctacagggaa ttgctattac 360
tcctgtacct tctgctcaat ttcctttcct catcccaaat aaatgccttg aaaaaaaaaa 420
aaaaaatga 429
```

<210> 10
<211> 429
<212> DNA
<213> Artificial sequence
<220>
<223> DNA enc.neutr.def.3 proprot.[H.sap.]with TPA sig.seq.,cod.opt.for expr.in mam.,nat.term.seq.
<400> 10

```
ATGGACGCCA TGAAGAGAGG CCTGTGCTGC GTGCTGCTGC TGTGCGGCGC CGTGTTCGTG  60
AGCCCCAGCG AGCCCCTGCA GGCCAGAGCC GACGAGGTGG CCGCCGCCCC CGAGCAGATC 120
GCCGCCGACA TCCCCGAGGT GGTGGTGAGC CTGGCCTGGG ACGAGAGCCT GGCCCCCAAG 180
CACCCCGGCA GCAGAAAGAA CATGGACTGC TACTGCAGAA TCCCCGCCTG CATCGCCGGC 240
GAGAGAAGAT ACGGCACCTG CATCTACCAG GGCAGACTGT GGGCCTTCTG CTGCTGAGCT 300
TGCAGAAAAA GAAAAATGAG CTCAAAATTT GCTTTGAGAG CTACAGGGAA TTGCTATTAC 360
TCCTGTACCT TCTGCTCAAT TTCCTTTCCT CATCTCAAAT AAATGCCTTG TTACAAAAAA 420
AAAAAATGA 429
```

<210> 11
<211> 300
<212> DNA
<213> Artificial sequence
<220>
<223> DNA enc.neutr.def.1 proprot.[H.sap.]with TPA sig.seq.,cod.opt.for expr.in mam.,stop-codon
<400> 11

```
atggacgcca tgaagagagg cctgtgctgc gtgctgctgc tgtgcggcgc cgtgttcgtg  60
agccccagcg agcccctgca ggccagagcc gacgaggtgg ccgccgcccc cgagcagatc 120
gccgccgaca tccccgaggt ggtggtgagc ctggcctggg acgagagcct ggcccccaag 180
caccccggca gcagaaagaa catggcctgc tactgcagaa tccccgcctg catcgccggc 240
gagagaagat acggcacctg catctaccag ggcagactgt gggccttctg ctgctgataa 300
```

<210> 12
<211> 300
<212> DNA
<213> Artificial sequence
<220>
<223> DNA enc.neutr.def.3 proprot.[H.sap.]with TPA sig.seq.,cod.opt.for expr.in mam.,stop-codon
<400> 12

```
atggacgcca tgaagagagg cctgtgctgc gtgctgctgc tgtgcggcgc cgtgttcgtg  60
agccccagcg agcccctgca ggccagagcc gacgaggtgg ccgccgcccc cgagcagatc 120
gccgccgaca tccccgaggt ggtggtgagc ctggcctggg acgagagcct ggcccccaag 180
caccccggca gcagaaagaa catggactgc tactgcagaa tccccgcctg catcgccggc 240
gagagaagat acggcacctg catctaccag ggcagactgt gggccttctg ctgctgataa 300
```

1

## Description

The invention (embodiments) relates to the field of medicine, pharmacology, biotechnology, molecular biology, genetic engineering and can be used for analgesia.

It is important to recognize that pain is not just an indicator of the underlying disease or damage process, it is an independent problem, causing great damage to individuals and society in whole. To improve the quality of life pain relief as such should be the therapeutic goal. A promising approach to the treatment of pain, which is proceeded to currently, - the identification of the mechanism and implementation of targeted (aimed at specific molecules) pharmacological therapy [WHO Normative Guidelines on Pain Management, Geneva June 2007].

Peptide amides [US4459225 (A)], phenylhydrazone derivatives are known to be used as anti-inflammatory or analgesic agents [EP0952159 (B1)], synthetic peptide amides - for the prevention and treatment of pain [RU2500685C2]. A family of peptides is known to be used possessing analgesic activity, of the general formula A-B-Tyr-Pro (DPro, dHPro, DdHPro, DLdhPro, Hyp)-C-X [WO2008020778 (A1)], a derived peptide is known to be used which is administered subcutaneously or orally possessing analgesic or anti-nociceptive activity [US2008009448 (A1)].

The disadvantage of chemically synthesized peptides and their derivatives is the ability to obtain a mixture of the desired compounds with enantiomers which may lead to unpredictable results. The concept of enantiomeric plays an important role in pharmaceutics because different enantiomers of drug substances usually have different biological activity. The use of living systems for obtaining peptide molecules allows to obtain the molecules of natural structure, a homogeneous mixture.

A recombinant plasmid is known, for bacterial expression, containing a homologous regulon or heterologous DNA, the least may encode a polypeptide with analgesic effect [SK278170 (B6)]. A mono-coupling of the indole with a new structure is known containing deep macrogenomic gene cluster, synthesized in *Escherichia coli* cells [CN102477436 (A)]. A polypeptide is known from the sea anemone *Heteractis crispa,* which has strong analgesic effect [RU2368621C1, RU2404245C1], and its preparation method [RU2415866C1]. Peptides are known with a large number of bridging ties, isolated from *ACTINOMADURA NAMIBIENSIS,* for the treatment of neuropathic pain caused by inflammation [RU2498995C2]. However, due to the fact that these molecules are derived from organisms that are distant from a human, their effect must be studied very carefully before use in a human.

A hybrid protein on the basis of conotoxin (snail poison) MVó A and Trx is known, which can be used in the preparation of pain meds for the last stages of cancer and AIDS, post-operative pain, burns, etc. [CN1487085 (A)]. A method of obtaining conotoxin using baculovirus and insect cells or insects is known [CN102876683 (A)]. Methods of analgesia and improved opiate analgesia introduction with omega-conopeptides TVIA (SNX-185) or MVIIA (SNX-111) introduction [EP0625162 (B1)], methods of analgesia using alpha conotoxins [WO2014023129 (A1), CN102286079 (B), CN103374066 (A)], Lt7b conotoxin [CN102628048 (A)] are known. It is known to use omega-conopeptide for the production of medicament for inhibiting the progress of neuropathic pain [EP1336409 (B1)]. A Scorpio polypeptide is known having analgesic and antitumor activity [US7592309 (B2), CN101591668 (A)], and a method of its application [US7592309 (B2) and CN101591668 (A)], a scolopendra *Scolopendra subspinipes mutilans* one - mu-SLPTX-Ssm6a - having an analgesic effect [CN102977201 (A)]. Peptides of the venom of the spider *Grammostola spatulata,* with analgesic effect [US5776896 (A)], a peptide HWAP-I of a Chinese tarantula [US6670329 (B2)] are known. Peptide molecules from snake venom and their derivatives, homologs, analogs and mimetics are known capable of inducing analgesia or alleviating pain alone or in combination with other analgesic molecules [US6613745 (B1), US7902152 (B2)], and also a shortened neurotoxin of the sea snake *Lapemis hardwickii* is known for analgesia [US7294697 (B2)].

The disadvantage of that group of inventions is that the use of toxins, even pharmaceutically pure, is still a risk - in case of overdose up to death.

A new polypeptide is known to be used - a subunit 9.01 [CN1345751 (A)], subunit 12.65 [WO0212310 (A1)], subunit 9.46 [WO0204504 (A1)], subunit 11.44 [CN1352195 (A)] of human G-protein for analgesia. Polypeptides are known based on a G-protein receptor HFIAO41 [US2001016336 (A1)], HLYAZ61 [EP0837128 (A2)], HUVCT36 [US5912335 (A)], H7TBA62 [US5955309 (A)], org10 [US2003162945 (A1)], org11 [WO0200725 (A2)] and also belonging to the family IGS4 [AU779993 (B2), US6998255 (B1)]. Variants of a spliced G-protein receptor induced by Epstein-Barr EBI 3 are known [US5874252 (A)].

Methods of treatment of pain caused by bone cancer, osteoarthritis, postoperative pain are known by administering of antagonist of nerve growth factor (antibodies) [RU2389509C2, RU2429013, RU2338555C2], partners of specific binding with this molecule are known [RU2406728C2]. It is shown that molecules capable of inhibiting the binding between TrkA receptor and NGF can be used as analgesics with a prolonged effect [RU2427387C2]. Antibodies against nerve growth factor are known having increased stability in vivo [RU2011149263A], high affinity for NGF [RU2473564C2], and a dosage form is known of a humanized antibody against the growth factor of nervous tissue [RU2427387C2].

Known methods of treatment of:

- pain and inflammation - using a peptide [RU2011105280A], by separation of the interaction between CRMP-2 and CaV2.2, resulting in the absence of activation of N-type calcium channel (CaV2.2) [WO2012009075 (A1)], and by the separation of the interaction of LI-CAM, ankyrin and voltage-dependent calcium channels the damage of axons is treated, neurotransmitter release and pain transmission are inhibited, and a calcium pump in neurons is blocked [US7737250 (B2)], an analog of a peptide toxin of Nav1.7 sodium channel for analgesia is known [US2013296247 (A1)].

- pain and inflammation in neuronal tissue using antagonists of IL-31, a monoclonal humanized antibody or a chimeric antibody [RU2440130C2], a prostatic acid phosphatase ("PAP") or its active variant, fragment or derivative [WO2009064497 (A1)],
- neuropathic pain using antibodies against CCR2 [RU2011110169A], neuropathic pain with the use of peptides derived from prosaposin [RU2007119313A], a peptide containing the amino acid sequence of Thr-R1-Lue-Ile-Asp-Asn-Asn-Ala-Thr-Glu-Glu-Ile-Leu-Tyr, where R1 is D-alanine, affecting neurodegenerative disorder or breach of myelination [RU2266129C2], proteins with zinc-finger domain associated with the regulatory domain, that can activate or suppress expression of a target gene associated with neuropathic pain (VR1, NaV1.8, and TrkA) [US8466267 (B2)].
- neuropathic or central sensibilisation pain, using a sequence of an isolated gene, which is regulated in the spinal cord of a mammal in response to mechanistically different first and second models of neuropathic or central sensibilization pain [US2003108906 (A1), US2003134301 (A1), US2003138803 (A1), US2004058326 (A1)],
- chronic pain with the use of the TORC polypeptide [RU98118091A], histogran and its chemically stable analog [CA2219437 (A1)], linear, cyclic histogranic peptides and pseudopeptides based on them [US6566327 (B1)], a hybrid protein containing IL-10 and IL-4 [WO2013070076 (A1)], polypeptides on the basis of IL-10 [US7261882 (B2)],
- acute and chronic pain (different forms of pain) using IL-13-binding protein [RU2472807C2], IL-12/p40 binding proteins [RU2012124438A], IL-12/p41-binding proteins [RU2008103312A], IL-17-binding proteins [RU2011140335A], prostaglandin E2-binding proteins [RU2011104228A], immunoglobulins with dual variable domain [RU2515108C2], immunoglobulins with dual variable domain against prostaglandin E2 [RU2011104348A], struggle with chronic or acute pain with the use of EE3-family proteins [RU2004114989A],
- visceral pain using a polypeptide that increases guanylate cyclase activity of receptors [RU2433133 (C2)],
- neuropathic pain, osteoarthritis pain, inflammatory pain using IL-1-binding proteins [RU2012154210 A], inflammatory processes of arthritis and other TNF-a mediated disorders or pathophysiological mechanisms, in particular various forms of pain, with the use of stable and soluble antibodies - inhibitors of TNF-a [RU2415151C2], arthritis pain, with the use of a humanized antibody - antagonist of CGRP [RU2467765C2], pain induced by at least one anticancer agent, with the use of at least one botulinum toxin or peptide based on it [RU2483747C2], including in conjunction with opioid derivative [RU2434637C2], pain with the use of PAM associated with Myc protein [RU2345785C2], with the use of Galanin receptor-like GPCR polypeptide [US2004053244 (A1), US2003073115 (A1), WO0168843 (A1)], kontulakin-G and its deglycosylated form [US6696408 (B1), US6525021 (B1)], with the use of peptide inhibitors of protein kinase C [US7939493 (B2)], visceral pain by administering antibodies-antagonists directed against a peptide associated with calcitonin gene [RU2012106468 A],
- abdominal pain, with the use of peptides, preferably from *Lactobacillus rhamnosus* [RU2011136454 A].

Methods of reducing pain sensitivity in physiological and pathophysiological conditions (for example, in allodynia and hyperalgesia) are known, especially of pain perception that is associated or mediated by mechanical sensitivity via TRPA1, using a TRPA1 antagonistic antibodies, preferably monoclonal antibodies [RU2430750 C2]. Analgesic/ anti-inflammatory agents are known selected from the following classes of receptor antagonists, receptor agonists and enzyme inhibitors, each class acting by different molecular mechanisms of action on pain and suppression of inflammation: (4) bradikinin receptor antagonists (peptides), antagonists of calcitonin gene-mediated peptide (CGRP) receptor (alpha-CGRP-(8-37), (8) antagonists of interleukin receptor (Lys-D-Pro-Thr) [RU2180852 C2], of beta-cellulin (BTC) protein [JP2000198744 (A)]. Methods are known of reduction of the pain of CRF2R modulated disorder using agonists of corticotropin-releasing factor (CRF 2R) receptor [RU2385878C2], treatment of pain with the use of a peptide having properties of amilin [RU2385878C2], of NOP peptide agonist [RU2012106820A], of peptides containing two and more amino acid residues of the 11-28 fragment of vasoactive intestinal peptide (VIP), delivered in the CNS [WO9104041 (A1)], of a derived peptide on the basis of calcitonin [US5446026A], human polypeptides CGRP-RCF [US5710024 (A), WO9803534 (A1)], polypeptides - humanized CGRP receptor, including for the treatment of headache, chronic headache of tension, severe paroxysmal headache with periodic recurrence, prevention of migraines [US7193070 (B2)], polypeptides - new spliced options of human 11cb [US6033872 (A)], agonists and antagonists of the latter protein [WO9928492 (A1)], receptors with seven transmembrane domains [US5824504 (A), US6277960 (B1), US6277977 (B1), US5955308 (A), US6221627 (B1), US2002106766 (A1), US5994098 (A), US6174994 (B1), US6037146 (A), US5874243 (A)], a peptide having phosphodiesterase activity [WO0166716 (A1)], polypeptides - human protein kinases hYAK3 and HOACF72 [EP0870825 (B1) and US5972606 (A), respectively].

A hybrid protein based on brain neurotrophic factor is known produced using prokaryotic soluble protein expression system [CN1978466 (B), WO9942480 (A1)]. Polypeptides are known based on receptor of neurotensin of type 2 for the treatment of pain [US6008050 (A)]. A conjugate of neurotensin or its analogues with therapeutic peptide is known, which can be used to induce hypothermia or analgesia [WO2010063122 (A1)]. Conformationally optimized analogues of alpha-melanotropin are known, which have a specific effect on the CNS [US4649191 (A)]. A synthetic peptide for modulation of the release of the neurotransmitter similar to SNARE is known [KR20090041066 (A)].

Peptides are known with multiple functions, including analgesia, such as peptides with a large number of bridging, extracted from *Actinomadura namibiensis* [RU2010144778A], a peptide of structure DGSVVVNKVSELPAGHGLNVNTLSYGDLAAD [RU2508296C2], a peptide modulator of purinergic receptors PT1 [RU2422459C1], dipeptides containing a 2-thioacyl group on an N-terminal amino acid residue as inhibitors of vasopeptidase [RU2298559C2], thymus-specific protein [RU2398776C2], a polypeptide improving calcium metabolism [JPH03178993 (A)] as well as prodrug compositions with a high degree of penetration based on peptides and compounds related to peptide [RU2011149796 A].

A combined approach to analgesia is known using a synergistic combination of a selective inhibitor of the neuronal norepinephrine transporter and an analgesic in the therapeutic treatment of vertebrates, including humans, for anesthesia or for the prevention or relief of pain [US 8188048 B2]. Peptides-analogues of dynorphin are known that, when co-administered with a narcotic or analgesic, reinforce the latter, including enkephalins and beta-endorphin analogues [EP0096592B1].

Methods are known of increasing the pharmacological activity of the substances with oral and parenteral administration of the compositions, with the use of the peptide Tyr-Gly-Gly-Phe-Met bound with a carrier group, thus forming a precursor of a drug used in the treatment or relief of pain [AU2002228260 (B2)].

The use of one highly effective agent for analgesia, simple and cheap to produce, is a more cost-effective option.

A use of defensin peptide Hnp-1 is known, one or in combination with neuropeptide M, as a therapeutic agent for the prophylaxis and/or treatment including peripheral vascular diseases, including anesthesia, spreading pain, causalgia (burning pain) [WO 2009/043461 A1].

The use of molecules of protein nature, which were not thought of as inducers of the immune response, including antibody, can be complicated by these very effects, which may lead to side effects, for example, associated with the formation of autoantibodies.

A use of small interfering RNA and of a short hairpin RNA is known for gene knockdown of NR1 subunit of N-methyl-D-aspartate subcutaneous receptor to reduce the inflammatory pain or intolerable chronic pain, especially chronic clinical pain and pain caused by burns [US8372817 (B2), US8575330 (B2), US8361985 (B2)]. RNA interfering agent is known for the treatment of chronic pain [WO2013126963 (A1)]. However, the preparations of RNA are quickly destroyed outside of the body, special storage conditions are required, which sets industrial applicability of drugs based on RNA a question.

A method of long-term analgesia is known in which the patient is injected with myogenic cells, in which from a corresponding DNA a peptide is synthesized that activates an opioid receptor or mediates the binding of substance P to the receptor [US7166279 (B2)]. However, the implementation of this method is quite complicated and is associated with many risks.

Methods of treatment of depression and pain are known, the agent may be a protein, RNA or DNA [WO2013177484 (A1)]. Lentiviral vector for the treatment of pain containing G-protein of rabies virus [EP1425403 (B1)], adenovirus encoding IL-24 [CN101518655 (A)], including for pain relief when cancer are known. Oncostatin M is known, or its homologue, synthesized from a vector for transfer of genes: lentivirus, retrovirus, Sendai virus, adenovirus and adeno-associated virus, including for the treatment of pain [JP4803789 (B2)]. Treatment of allodynia, hyperalgesia, spontaneous pain and phantom pain using kometin is known - polypeptide, which can be delivered as a polypeptide, or by introduction of expression vector for expression of kometin, a cell line, transformed or transfected with the protein and a capsule containing the cells [WO2013034157 (A1)]. Viral vectors have disadvantages, including the fact that they are expensive and can cause inflammatory response that precludes repeated administration of the vector. Viral vectors can replicate, which reduces the degree of control over expression of the targeted protein, which in turn is not always desirable and applicable, particularly in relation to analgesia.

A recombinant DNA-vaccine based on a pVAX1 vector is known, targeted to a tumor, including for the treatment of pain in oncology that contains a gene encoding cyclooxygenase-2 (COX-2), murine ubiquitin Mubi, as well as ISS immunostimulatory sequences of DNA (pVAX1-mUbi-ISS-COX-2-ISS) [CN101648011 (A)]. However, this design is aimed to induce an immune response, cytotoxic, for which elements are introduced that lead to and increase immunogenicity. A pharmaceutical composition is known, which contains a vector, including a plasmid DNA, and a pharmaceutically acceptable excipient or adjuvant to alleviate, prevent or treat pain, the vector contains a nucleotide sequence containing at least one area modulating the expression of the VR1 receptor [US2006154886 (A1)]. In this case analgesia is via blocking of a receptor associated with an ion channel - a nonspecific cation conductor. The use of plasmid DNA bearing a gene from which in mammal cells defensin is synthesized as a means of inducing analgesia will also help to minimize the possibility of the development of pathogens in the body: usually when the pain of any etiology the state of health worsens, and the body's ability to resist infections decreases, and as a result, after, for example, surgery, antibiotics can be prescribed, and the use of natural antimicrobial peptide will allow to combine analgesia and a protective effect, as a result, it is possible to refuse to take antibiotics.

Close analogues of the proposed invention are the inventions described in the patent CN101648011 (A) and the application for the invention US2006154886 (A1), which disclose analgesics in the form of plasmid DNA.

It is shown that when intramuscular injection of DNA, expression vector is absorbed by muscle cells, and expression of an encoded gene in the input vector is performed [J. A. Wolff et al., Science 247, 1465 (1990); G. Ascadi et al., Nature 352, 815 (1991)]. It is shown that the plasmids are maintained as episome and do not replicate. A constant expression has been demonstrated after intramuscular injection also in the skeletal muscles of rats, fish and primates, and cardiac muscle of rats [H. Lin et al, Circulation 82, 2217 (1990); R. N. Kitsis et al, Proc. Natl Acad. Sci. (USA) 88, 4138 (1991); E. Hansen et. al, FEBS Lett. 290, 73 (1991); S. Jiao et al., Hum. Gene Therapy 3, 21 (1992); J. A. Wolff et al, Human Mol. Genet. 1,363 (1992)]. The use of other technologies of delivery of a plasmid DNA into cells of various tissues is known, for example, of a needleless syringe, allowing for the delivery into cells of various tissues of living animals [Furth et al., Analytical Biochemistry, 205, 365-368, (1992)].

It is shown that normally muscle fibers do not express MHC antigens, but during the inflammation associated with the infection, or in the presence of interferon gamma muscle fiber is capable of producing antigens in the complex with the first class MHC or even second class [MECKERT, P. C, HONTEBEYRIE-JOSKOWICZ, M., CHAMBO, J., LEVIN, M., and LAGUENS, R. P. (1991). Trypanosoma cruzi: Aberrant expression of class II major histocompatibility complex molecules in skeletal and heart muscle cells of chronically infected mice. Exp. Parasitol. 72, 8-14; Hohlfeld and ENGEL, A. G. (1984) The immunobiology of muscle. Immunol. Today 15, 269-274.; MANTEGAZZA, R. & BERNASCONI, P. (1994). Cellular aspects of myositis. Curr. Opin. Rheumatol. 6, 568-574, Hartikka J, Sawdey M, Cornefert-Jensen F, Margalith M, Barnhart K, Nolasco M, Vahlsing HL, Meek J, Marquet M, Hobart P, Norman J, Manthorpe M. An improved plasmid DNA expression vector for direct injection into skeletal muscle. Hum Gene Ther. 1996 Jun 20;7(10):1205-17], in this connection, the introduction of a plasmid DNA in which the injury to the muscle is minimized is preferred.

It is shown that even being immunogenic in tests on laboratory animals, in humans formulations based on a plasmid DNA are not immunogenic [Saade F, Petrovsky N. Technologies for enhanced efficacy of DNA vaccines. Expert Rev Vaccines. 2012 Feb;11(2):189-209. doi: 10.1586/erv.11.188]. The researchers also failed to find evidence of pathological changes after the repeated injections of DNA in mice or rabbits [Parker SE, Borellini F, Wenk ML, et al. Plasmid DNA malaria vaccine: tissue distribution and safety studies in mice and rabbits. Hum. Gene Ther. 1999;10(5):741-758.]. In 2007, in the U.S. FDA guidance on DNA vaccines concluded that preclinical studies are not required to assess the effect against autoimmune diseases.

A plasmid DNA should contain elements essential for organisms of its maintenance and use, together with appropriate regulatory sequences. Regulatory sequences are nucleotide sequences that can affect gene expression at the level of transcription and/or translation, as well as mechanisms ensuring the existence and maintenance of the functioning of the plasmid DNA.

For prokaryotic system origin of replication and a marker gene are essential. Bacterial elements of the plasmid DNA are not to negatively influence expression in mammalian cells and cause side effects from the use of the plasmid DNA. In the literature, there is evidence that the use of a gene of resistance to ampicillin as a marker gene may be undesirable in connection with the development of the reaction in patients on ampicillin, but the authors consider such effects associated with a low quality of purification of a plasmid DNA, but not by the element itself.

Essential elements of plasmids for use in mammals are a promoter, an mRNA leader sequence, a termination sequence.

A promoter is an important component of the plasmid, which triggers the expression of a gene of interest. Classic promoters for plasmid DNA - components of drugs - is a human CMV / immediate-early or CMV-chicken-$\beta$ actin (CAGG) promoter. CMV promoters are used for the most DNA vaccines, as they mediate high levels of constitutive expression in a wide range of mammalian tissues [Manthorpe M, Cornefert-Jensen F, Hartikka J, et al. Gene therapy by intramuscular injection of plasmid DNA: studies on firefly luciferase gene expression in mice. Hum. Gene Ther. 1993;4(4):419-431] and do not inhibit the downstream reading. The increase in expression level is observed when changing the CMV promoter, for example, by the inclusion of HTLV-1R-U5 downstream from the cytomegalovirus promoter or using a chimeric SV40-CMV promoter [Williams JA, Carnes AE, Hodgson CP. Plasmid DNA vaccine vector design: impact on efficacy, safety and upstream production. Biotechnol. Adv. 2009;27(4):353-370]. Tissue-specific host promoters serve as an alternative to the CMV promoter, they allow to avoid constitutive expression of antigens in inappropriate tissues, which generally leads to lower immunogenicity [Cazeaux N, Bennasser Y, Vidal PL, Li Z, Paulin D, Bahraoui E. Comparative study of immune responses induced after immunization with plasmids encoding the HIV-1 Nef protein under the control of the CMV-IE or the muscle-specific desmin promoter. Vaccine. 2002;20(27-28):3322-3331].

The leader mRNA sequence also plays a big role. The Kozak sequence directly before the start codon ATG allows to increase the expression [Kozak M. Recognition of AUG and alternative initiator codons is augmented by G in position +4 but is not generally affected by the nucleotides in positions +5 and +6. EMBO J. 1997;16(9):2482-2492]. The presence of an intron in the plasmid downstream from the promoter can increase the stability of mRNA and enhance the gene expression.

The use of species-specific codons allows to increase the expression of the gene [Frelin L, Ahlen G, Alheim M, et al. Codon optimization and mRNA amplification effectively enhances the immunogenicity of the hepatitis C virus nonstructural 3/4A gene. Gene Ther. 2004;11(6):522-533].

Gene expression can be influenced by changing the terminating sequence, which is needed to preserve the stability of mRNA, for the proper termination of transcription and export of mRNA from the nucleus, including its shortening. Many of today's DNA vaccines use the sequence of the transcription terminator of bovine growth hormone [Montgomery DL, Shiver JW, Leander KR, et al. Heterologous and homologous protection against influenza A by DNA vaccination: optimization of DNA vectors. DNA Cell Biol. 1993;12(9):777-783]. Polyadenylation (polyA) is required for stabilization of the transcript. Changing of the polyA sequence can lead to the increase of gene expression level [Norman JA, Hobart P, Manthorpe M, Felgner P, Wheeler C. Development of improved vectors for DNA-based immunization and other gene therapy applications. Vaccine. 1997;15(8):801-803]. In pVAX1 (Invitrogen, Carlsbad, CA) a terminator region of bovine growth hormone contains a homopurine fragment, which is sensitive to the nuclease. It is shown that alternative polyA sequence can significantly improve the stability of the plasmid to a nuclease [Azzoni AR, Ribeiro SC, Monteiro GA, Prazeres DMF. The impact of polyadenylation signals on plasmid nucleases-resistance and transgene expression. J Gene Med. 2007;9:392-402]. The introduction of two stop codons allows to increase the efficiency of the transcription terminator.

The optimal plasmid design for the implementation of the function should combine the "bacterial" and "eukaryotic" elements with appropriate regulatory sequences to ensure a high number of copies per cell during the production process and a high level of expression in mammals [Saade F, Petrovsky N. Technologies for enhanced efficacy of DNA vaccines. Expert Rev Vaccines. 2012 Feb;11(2):189-209. doi: 10.1586/erv.11.188].

To create analgesics according to the present invention it is possible to use a plasmid DNA, as a minimum, standardly used for gene delivery and expression in the mammals, including humans, and also optimized on the above parameters, including those described in detail in Williams et al., 2009 [Williams JA, Carnes AE, Hodgson CP. Plasmid DNA vaccine vector design: impact on efficacy, safety and upstream production. Biotechnol. Adv. 2009;27(4):353-370]. In the FDA guideline (2007) it is stated that studies of the biodistribution of a substance after its introduction in the body can be waived for DNA vaccines produced by cloning of a new gene into a plasmid vector, for which acceptable biodistribution and integration profile are documented previously. In the WHO guideline (2007) it is stated that research on the biological distribution and conservation is required if there is no considerable experience of work with almost identical or similar product. In the EMEA guidance (2006) it is stated that the experience of work with the vector system will allow to optimize and focus on preclinical studies. Safety studies with the use of DNA vectors with different cloned genes showed similar biodistribution [Sheets RL, Stein J, Manetz TS, Duffy C, Nason M, Andrews C, Kong WP, Nabel GJ Gomez PL. Biodistribution of DNA plasmid vaccines against HIV-1, Ebola, Severe Acute Respiratory Syndrome, or West Nile virus is similar, without integration, despite differing plasmid backbones or gene inserts. Sheets RL, Stein J, Manetz TS, Duffy C, Nason M, Andrews C, Kong WP, Nabel GJ Gomez PL.Toxicol Sci. 2006 Jun;91(2):610-9. Epub 2006 Mar 28.] and toxicology [Sheets RL, Stein J, Manetz TS, Andrews C, Bailer R, Rathmann J, Gomez PL. Toxicological safety evaluation of DNA plasmid vaccines against HIV-1, Ebola, Severe Acute Respiratory Syndrome, or West Nile virus is similar despite differing plasmid backbones or gene-inserts. Toxicol Sci. 2006 Jun;91(2):620-30. Epub 2006 Mar 28]. For a plasmid DNA for use in mammals other than humans the requirements are less stringent, therefore it is possible to use a wider range of plasmids.

For DNA intended for human use, it may be useful to have the final DNA product in a pharmaceutically acceptable carrier or a buffer solution. Pharmaceutically acceptable carriers or buffer solutions are known from the art and include those described in various texts, such as Remington's Pharmaceutical Sciences.

The prototype of the embodiments of the invention is the invention disclosed in a patent application published as WO 2009/043461 A1: application of defensin HNP-1 for the treatment including pain.

Defensins constitute a large family of low molecular weight (3-5-kDa) cysteine-rich cationic peptides stabilized by several (usually three) disulfide bonds [Ganz, T. 2002. Immunology: Versatile defensins. Science 298: 977-979, Lehrer, R. I. and Ganz, T. 2002. Defensins of vertebrate animals. Curr. Opin. Immunol. 14: 96-102.], which are capable of killing a broad spectrum of pathogens, including various bacteria, fungi and enveloped viruses. In humans this family is represented by $\alpha$-subfamily (HNP) and $\beta$- subfamily (hBD) of defensins. Alpha-defensins are mostly represented in neutrophils and Paneth cells. HNP-1 and HNP-3 contain 30 amino acid residues in total, these peptides are identical to each other except for the substitution of alanine for asparagine in position 1. HNP-2 is a proteolytic product of HNP-1 and HNP-3, contains 29 amino acid residues (missing the first amino acid from the N-terminus). The presence of disulfide bonds ensures saving of the stability of the defensin molecules to numerous leukocyte and microbial proteinases and saving antibiotic properties in the center of inflammation and tissue destruction [O. Levy Antimicrobial proteins and peptides: anti-infective molecules of mammalian leukocytes. J. of Leukocyte Biology.2004; 76: 909-926]. For these molecules to be obtained in a correct spatial structure is quite challenging.

Genes encoding defensins form a cluster at the locus P22-23 on the 8th chromosome. HNP 1-3 are encoded by two genes HDEFA1 and HDEFA3. Each defensins gene contains several exons that encode prepropeptide.

First, defensins are synthesized in the form of precursors, as prepropeptides, the length of which is 94 amino acid residues. Prepropeptides contain the signal fragment (an average of 19 amino acid residues), anionic fragment (an average of 45 amino acid residues) and the "mature" peptide. In the result of a proteolytic cleavage in the endoplasmic reticulum from a prepropeptide a removal of the signal fragment and a prodefensin formation are performed (at average 75 amino acid residues). Subsequent maturation (cleavage of 45 amino acid residues) occurs in the mature neutrophils granules. $\alpha$-Defensins were also found in NK cells, B-lymphocytes, $\gamma\delta$ T-lymphocytes, monocytes/macrophages and epithelial cells [A. S. Budikhina, B. V. Pinegin. Defensins - multifunctional cationic peptides of the person. Immunopathology, Allergology, Infectology. 2008, No. 2:31-40]. The authors of the present invention at first demonstrated that the introduction of a plasmid DNA carrying the gene encoding HNP-1/ HNP-2/ HNP-3 leads to analgesia, moreover, in this case the increase of the level of beta-endorphin in the serum was observed.

The disadvantages of analogues and the prototype are shown directly after their description. The claimed invention (embodiments) is free from these disadvantages.

The technical result from the use of embodiments of the invention is expressed, firstly, in expanding the spectrum of analgesics. When poor tolerability or intolerance of analogues a representative of the proposed versions of the analgesic would allow analgesia, due to which the patient will have the opportunity to take actions that he could not, or did not want to carry out without analgesia, for example, to dare on a needed procedure, as well as improve the quality of life, for example, will get less discomfort than without the use of analgesic, or will be able to alleviate acute or chronic pain. This technical result is achieved by the use of analgesic according to the present invention (embodiments).

In addition, the technical result is to increase the duration of analgesia and is achieved by the plasmid DNA use, from which after the introduction into the body alpha human defensin HNP-1/HNP-2/HNP-3 is synthesized; and by the fact that the nucleotide sequence encoding the human defensin, contains elements that lead to mRNA stability and, consequently, increases the half-life time of mRNA, as a result, the protein synthesis from one molecule of mRNA is carried out more number of times, and also as a result the amount of protein synthesized increases; and also the fact that the nucleotide sequence of human alpha defensin is codon-optimized for expression in mammals, as a result the protein synthesis is more intensive. During implementation into practice this will allow to significantly reduce the amount of plasmid DNA introduced (in 10-50 times) in comparison with the doses currently used in a blighty and world practice in gene therapy.

In addition, the technical result is to increase the safety of analgesia. This technical result is achieved by the fact that the active ingredient is not a protein to which antibodies can be formed, which, when a molecule of the original organism is used, can cause serious side effects, but a plasmid DNA, which exists as an episome and does not integrate into the genome, from which the human protein is synthesized and then secreted, and as a result, a nonimmunogenic, safe use of human defensin for analgesia is performed. This technical result is also achieved by the fact that the protein synthesized in the organism from the plasmid DNA undergoes natural post-translational processing, and the correct folding of the protein due to cellular chaperones is also ensured. These modifications are difficult to achieve during the production of proteins, which can dramatically affect a number of their functions. This technical result is also achieved due to the fact that natural mechanisms of metabolism and catabolism of the active substance are used, without the formation of toxic products, due to the nature of the plasmid construct and the encoded protein, as well as the identity of the protein formed to the endogenous analogue. This technical result is also achieved by the fact that the plasmid DNA does not replicate after introduction into the mammalian organism, which allows to control the amount of a synthesized protein, and, accordingly, over analgesia. This technical result is also achieved by the presence of such regulatory sequences as the silencer and / or insulator in the plasmid DNA, in one embodiment of the invention, which allows also to control the amount of the protein synthesized, and, basically, the synthesis of the protein as such, and, respectively, analgesia: if necessary, there is an opportunity in a short period to stop or decrease the expression of the gene. In the latter variant, it is also possible to perform tissue-specific expression when necessary.

The technical result is also to simplify and reduce the cost of producing the analgesic by avoiding the complexities of production and the processes of protein preparations purification in vitro, due to the fact that the protein synthesis occurs in vivo. Production, purification and storage of DNA preparations are more economically profitable than of protein ones, since the former are more stable, they can be produced in large quantities and at lower cost.

It was shown that the change in the concentration of beta-endorphin in the blood plasma is directly dependent on the type of pain syndrome, its intensity and analgesia efficiency, which can serve as a criterion for assessing the effectiveness of analgesia [Beta-endorphin - an analgesic effectiveness marker for acute pain and chronic pain syndrome in cancer Patients / ZV Pavlova [and others] // Problems of Clinical Medicine. - 2007. - N1. - P. 36-40. - ISSN 1817-8359]. When the analgesic of the present invention was administered, an increase in the serum beta-endorphin level was observed (Fig. 3).

According to the temporal characteristics, two types of pain can be distinguished:

- acute pain - a new, recent pain that is inextricably bound with the damage that has caused it and, as a rule, is a symptom of a disease, disappears when the damage is eliminated [Eddy N.B., Leimbach D. J. // Pharmacol Exp Ther. - Mar; 107 (3): 385-93. - 1953] (including pre- and postoperative, post-traumatic, in burns, acute pain during childbirth, pain caused by spinal cord trauma, acute headache, pain in HIV/AIDS, sickle cell crisis, trigeminal neuralgia, pancreatitis and other pains in the digestive tract, with myocardial infarction and other major cardiac events, interventional pain (during diagnostic and therapeutic procedures), acute in chronic pain [WHO Normative Guidelines on Pain Management, Geneva June 2007], which lasts up to 2-3 months, and can irradiate, and
- chronic pain - continuing for a long period of time (more than 2-3 months), even after the cause has been eliminated, often acquires the status of an independent disease, for example, an inflammatory process [Eddy N.B., Leimbach D. J. // Pharmacol Exp Ther. - Mar; 107 (3): 385-93. - 1953], with a decrease in the effectiveness of analgesics observed. A chronic pain may be a chronic pain in malignant diseases (including pain in cancer patients, HIV/AIDS, in amyotrophic lateral sclerosis (ALS), multiple sclerosis, at the final stage of an organ failure, extended chronic obstructive pulmonary disease, enhanced congestive heart failure, parkinsonism) and chronic pain not associated with a malignant disease (chronic musculoskeletal pain such as back pain or lower back pain, in a chronic degenerative arthritis, osteoarthritis, rheumatoid arthritis, myofascial and rheumatic pains, chronic headache, migraine headache, bone pains; neuropathic pains (including pain in nerve compressions, injuries, pains after nerve injury and amputation), diabetic neuropathy, complex regional pain syndromes (type I and type II) and spasms of skeletal muscles, in post-herpetic neuralgia, chronic pains after surgical interventions; visceral pain (if stretching of hollow viscera and colics); and chronic pain in sickle-cell anemia [WHO Normative Guidelines on Pain Management, Geneva June 2007].

With the use of this analgesic the both described types of pain may be relieved and eliminated. As can be seen from the prior art, a plasmid analgesic, from which in mammalian cells human alpha defensin is synthesized, is unknown. The multiplicity and nature of the elements of a plasmid DNA require clarifying characteristics. The description of the invention, in the part "the summary of the invention", in the part "detailed description of the invention" explains "the formula of the invention" and includes a detailed description of essential elements of a plasmid DNA, which allows to implement the invention with all its embodiments, thereby achieving the described technical result.

Summary of the invention

A plasmid DNA is proposed for a transient expression in mammalian cells, represented by a skeleton that contains prokaryotic elements, origin of replication and a marker gene, and eukaryotic elements, a strong promoter, a leader mRNA sequence and regulatory sequences for these elements, at least one site to clone the gene of interest and at least one of site for at least one primer annealing for analysis of the content of the plasmid DNA, and a polynucleotide represented by a secretory sequence, a fragment encoding human alpha defensin HNP-1 or HNP-2 or HNP-3, codon-optimized for expression in mammalian cells, and a termination sequence. In one embodiment of the invention preproprotein is represented by SEQ ID NO:1 or SEQ ID NO:2 or SEQ ID NO:7 or SEQ ID NO:8. In one embodiment of the invention a polynucleotide is represented by SEQ ID NO:3 or SEQ ID NO:4 or SEQ ID NO:5 or SEQ ID NO:6 or SEQ ID NO:9 or SEQ ID NO:10 or SEQ ID NO:11 or SEQ ID NO:12.

Also a producer of such plasmid DNA is given based on a bacterial cell (embodiments) and an analgesic agent based on such a plasmid DNA in an effective amount, also containing a pharmaceutically acceptable excipient, for use in mammals, particularly humans (embodiments).

*Detailed description of the invention*

Plasmid DNA is the most economically advantageous to be produced in prokaryotic cells, mainly bacterial cells. In this regard, the plasmid DNA according to the present invention contains elements for maintenance and amplification, mainly in large quantities, in bacterial cells. Such essential elements are a bacterial origin of replication for maintenance in the cell with a medium, preferably a high number of copies per cell, and a marker gene for selection of a producer strain. Eligible origin of replication is pM1 (der.), ColE1 (der.) and F1, F1 and pUC, but is not limited to them. Eligible marker gene is a reporter gene or a gene of resistance to an antibiotic, e.g., ampicillin, kanamycin mainly, but not limited to them.

Plasmid DNA according to the present invention contains elements for effective functioning in mammalian cells.

Such an element is a promoter with corresponding regulatory sequences of the natural promoters with their regulatory elements (CaM kinase II, CMV, nestin, L7, BDNF, NF, MBP, NSE, p-globin, GFAP, GAP43, tyrosine hydroxylase, subunit 1 of a kainate receptor and subunit B of the glutamate receptor, and others) or synthetic promoters with regulatory sequences, to obtain the desired expression pattern (ratio of length and expression level) of the target gene at the transcriptional level.

Possible regulatory sequences relative to the promoter:

- enhancer, to increase the expression level via improvement of interaction between RNA polymerase and DNA,
- insulator, for modulating the function of the enhancer,
- silencer, or fragments thereof, to decrease the level of transcription, for example, for tissue-specific expression,
- 5' noncoding region upstream of the promoter, including intron.

Plasmid DNA according to the present invention contains at least one of the above-mentioned regulatory sequences, depending on the variant of plasmid DNA, based on the choice of promoter and the desired parameters of the expression of the target gene. Based on the existing level of technology, and the known and obvious variants of such elements and their use, plasmid DNA according to the present invention may contain any combinations meeting the mentioned above conditions, in which the synthesis of alpha defensin HNP-1 or HNP-2 or HNP-3 in mammalian cells is performed from the plasmid DNA. When a silencer is used, or insulator, as part of the design, it is possible to regulate the expression of a target gene, the gene of the described human alpha defensin HNP-1 or HNP-2 or HNP-3 (embodiments).

Other regulatory sequences:

- noncoding region downstream from the promoter, including intron, to increase the stability of mRNA and the expression of the target gene.

Plasmid DNA according to the present invention in one embodiment further comprises such a regulatory element.

Plasmid DNA according to the present invention contains such an important element as a leader mRNA sequence containing Kozak sequence directly before the start codon ATG.

Plasmid DNA also contains a site, preferably sites, different, for cloning of a target gene, for the correct orientation of the target gene in the plasmid DNA, and a site, preferably sites, for primers annealing for its sequencing.

Plasmid DNA also contains a termination sequence containing sequentially a stop codon, the 3' noncoding region with signal and polyadenylation site, stop codon, with the help of which mRNA remains stable, and the proper termination of transcription and export of mRNA from the nucleus are performed. The terminating sequence is a native sequence, i.e. inherent from the target gene, or other, more powerful, which is represented, for example, by the termination sequence of bovine growth hormone (BGH), but is not limited to such, and in the second embodiment may contain an additional stop codon before the 3' noncoding region. Based on the existing level of technology, and the known and obvious variants of such elements, plasmid DNA according to the present invention may contain any terminating sequence meeting the above conditions, in the presence of which the synthesis of alpha defensin HNP-1 or HNP-2 or HNP-3 is performed in mammalian cells from the plasmid DNA.

Plasmid DNA also includes a native or a heterologous secretory sequence, codon-optimized for mammals. In one embodiment of the invention it contains, for example, TPA (tissue-type plasminogen activator isoform 1 preproprotein [Homo sapiens], NCBI Reference Sequence: NP_000921.1) secretory sequence, but is not limited to such. The advantage of using TPA secretory sequence is a vast previous clinical experience, and also that its high performance is demonstrated in relation to the expression of the secreted protein from a variety of target genes.

Plasmid DNA also contains a fragment encoding human alpha defensin HNP-1 or HNP-2 or HNP-3, codon-optimized for expression in mammalian cells.

Codon optimization is carried out to increase the expression of the target gene by increasing the efficiency of reading information from mRNA on the ribosomes.

Plasmid DNA can additionally include initial for cDNAs of these genes elements that contribute to the stability of this mRNA, such as a terminating sequence (3' noncoding region containing the signal and the site of polyadenylation, and also the signal of termination of the transcription - stop codon). Accordingly, in this case analogous elements in the skeleton of the plasmid DNA do not exist or do not function.

Plasmid DNA for the delivery of the gene causing analgesia, combines such properties of DNA vaccines based on a plasmid DNA as a high level of expression of a gene of interest in mammalian cells, and such properties of a vector for a gene therapy, as a lack of immunogenicity and a duration of the gene expression, however, for example, by increasing the stability of mRNA. Such DNA does not integrate into the genome and is not replicated in mammalian cells.

Suitable vectors for expression in mammalian cells are represented by known to a person averagely skilled in the art and described in the literature [Hartikka J, Sawdey M, Cornefert-Jensen F, Margalith M, Barnhart K, Nolasco M, Vahlsing HL, Meek J, Marquet M, Hobart P, Norman J, Manthorpe M. An improved plasmid DNA expression vector for direct injection into skeletal muscle. Hum Gene Ther. 1996 Jun 20;7(10):1205-17 etc.] as well as plasmids, which can be created by a person averagely skilled in the art using the guidelines on the elements of vectors ["Cloning Vectors", ed. Pouwls et al., Elsevier, Amsterdam - New York-Oxford, 1985, ISBN 0 444 904018, Williams JA, Carnes AE, Hodgson CP. Plasmid DNA vaccine vector design: impact on efficacy, safety and upstream production. Biotechnol Adv. 2009 Jul-Aug;27(4):353-70. doi: 10.1016/j.biotechadv.2009.02.003. Epub 2009 Feb 20. Review and others]. The preferred plasmid DNA for use in humans are vectors tested on humans containing the above-described elements with corresponding regulatory sequences, feasibly modified to match the stated criteria, which allows to reduce the number of required studies for the registration of a drug. However, the use of other plasmid DNAs containing the required described elements is possible.

The sequence of the elements described in the plasmid DNA is understandable to an expert in this field.

A producer of the plasmid DNA is given based on bacterial cells (based on cells mainly of *Escherichia coli, Streptomyces, Bacillus, Pseudomonas,* but not limited to such). It is clear to the person skilled in the art that using a plasmid DNA according to the invention and a bacterial cell, e.g., commercial, but it not limited to such, it is possible to create a producer of the plasmid DNA, for example, by standard methods, e.g., transfection, electroporation or particle gun. To reduce the probability of mutations due to methylation of a plasmid DNA it is preferable to use a strain of a microorganism not containing methylase in the genome.

Also an analgesic agent is given based on the described plasmid DNA, in an effective amount, also containing a pharmaceutically acceptable excipient, for use in mammals, particularly in humans. This pharmaceutical composition is to treat, alleviate and/or prevent pain, in particular, acute or chronic pain, disorders of sensitivity.

The authors of this invention conducted laboratory tests confirming the possibility of implementing the described group of inventions. The results obtained are illustrated by examples (1-3) and Fig.1-3.

*Brief description of the drawings.*

Fig. 1. The results of the test "hot plate". The x-axis is the latency time of licking of the front and rear legs, the ordinate axis is the time after administration of the test substance. Legend: 1 - negative control (saline injected), 2 - pVAX1seq3, 3 - pVR1012seq4, 4 - pVR1012seq5, 5 - pVAX1seq6, 6 - pVR1012seq9, 7 - pVAX1seq10, 8 - pVAX1seq11, 9 - pVR1012seq12, 10 - pcDNA3.1+ seq11, 11 - pcDNA3.1+ (var)seq12, 12 - analgin (50 mg/kg), 13 - morphine hydrochloride (10 mg/kg), 14 - pVAX1, 15 - pVR1012, 16 - pcDNA3.1+, 17 - pcDNA3.1+(var). Plasmid DNA was injected in an amount of 5 mg/kg.

Fig. 2. The results of a study of different doses of plasmid DNA pVAX1seq3 in the test "hot plate". The x-axis is the latency time of licking of the front and rear legs, the ordinate axis is the time after administration of the test substance. Legend: 1 - negative control (saline injected), 2 - pVAX1seq3 1 mg/kg, 3 - pVAX1seq3 5 mg/kg, 4 - pVAX1seq3 10 mg/kg, 6 - analgin (50 mg/kg), 7 - morphine hydrochloride (10 mg/kg).

Fig. 3. The results of the study of changes in the concentration of beta-endorphin in the serum of mice. The x-axis is time after administration of the test substance, the ordinate axis is the concentration of beta-endorphin in the serum. Legend: 1 - negative control (saline injected), 2 - pVR1012, 3 - pVR1012seq4. Plasmid DNA was injected in an amount of 5 mg/kg.

**Example 1. Obtaining of plasmid DNA encoding alpha defensin HNP-1 or HNP-2 or HNP-3.**

1.1. A chemical synthesis of DNA characterized by SEQ ID NO:3 - SEQ ID NO:6 and SEQ ID NO:9 - SEQ ID NO:12, optionally flanked by restriction sites, with the addition of a Kozak sequence, was performed. For SEQ ID NO:3 and SEQ ID NO:10 restriction sites NheI (5'), BamHI (3') were used, for SEQ ID NO:4 and SEQ ID NO:9 restriction sites SalI (5'), KpnI (3') were used, for SEQ ID NO:5 and SEQ ID NO:12 restriction sites SalI (5'), BamHI (3') were used, for SEQ ID NO:6 and SEQ ID NO:11 restriction sites NheI (5'), ApaI (3') were used.

1.2. Cloning of the synthesized gene into pVAX1 plasmid (Invitrogen) and pVR1012 (Vical) was performed. Also cloning of the sequence SEQ ID NO:11 was performed in the vector pcDNA3.1+ (Invitrogen) at restriction sites NheI (5'), ApaI (3'). Also a vector pcDNA3.1+ incapable of the expression of neomycin was received, due to the restriction of this vector with NsiI restrictase, in the region of the SV40 promoter (-71 bp). SEQ ID NO:12 was cloned in the resulting vector at restriction sites NheI (5'), ApaI (3').

1.3. For the ligation reaction 3 $\mu$l of the synthesized DNA solution, 1 $\mu$l of the prepared vector solution, 5 $\mu$l of buffer for ligation $\times$2 and 1 $\mu$l of T4-ligase were taken. The reaction was performed at +20°C for 2 hours.

Thereafter, the mixture was warmed up at +95°C for 10 min and purified from salts by dialysis using nitrocellulose filters with pore diameter of 0.025 $\mu$m (Millipore, USA). Dialysis was performed against a solution containing 0.5 mM EDTA in 10% glycerol, for 10 min.

The following plasmid DNAs were obtained: pVAX1seq3, pVR1012seq4, pVR1012seq5, pVAX1seq6, pVR1012seq9, pVAX1seq10, pVAX1seq11, pVR1012seq12, pcDNA3.1+ seq11, pcDNA3.1+ (var)seq12.

1.4. Then the cells of *E.coli* strain DH10B/R (F-mcrA, Δ(mrr-hsdRMS-mcrBC), φ80dlacZΔM 15, ΔlacX74, deoR, recA1, endA1, araD139, Δ(ara,leu)769, galU, galKλ-, rpsL, nupG) were transformed with the obtained plasmid DNA by the method of electroporation using electroporator MicroPulser (BioRad). This strain does not contain methylase, which helps minimize the possibility of mutations occurrence in DNA, including in gene cloned into the plasmid, sustained in this strain. 1 μl of dialyzed ligase mixture was added to 12 μl of the competent cells and placed between the electrodes of the poration unit and treated with a current pulse.

After transformation, cells were placed in 1 ml of SOC-medium (2% bacto-tripton, 0.5% yeast extract, 10 mM NaCl, 2.5 mM KCl, 10 mM $MgCl_2$, 10 mM $MgSO_4$, 20 mM glucose) and incubated for 40 min at +37°C.

1.5. Identification of clones of *E. coli* cells containing the plasmid DNA was performed on the selective medium containing LB-agar, 50 μg/ml of kanamycin, or ampicillin (for plasmid DNAs based on pcDNA3.1+).

Plasmid DNA was isolated from the grown clones. Isolation of the plasmid DNA was performed using Wizard Minipreps DNA Purification System (Promega, USA). Purified recombinant plasmid DNA was verified by sequencing.

1.6. Sequencing of the cloned fragments was performed by the method of Sanger using a set of Applied Biosystems BigDye® Terminator (BDT) v3.1 Cycle Sequencing Kit (Applied Biosystems, USA) according to the attached instructions. For the labeling of the reaction products fluorescent dye-labeled ddNTP were used, each ddNTP corresponding to the dye. For sequencing unlabeled plasmid-specific primers were used. A PCR reaction was conducted, then the reaction mixture was purified from free labeled ddNTP using instructions in the kit BigDye X-Terminator Purification Kit (Applied Biosystems, USA), and the sequencing reaction products were separated using capillary sequencer Applied Biosystems 3500/3500xL Genetic Analyzer (Applied Biosystems, USA) and reagent 3500/3500xL Genetic Analyzer Polymer "POP-6™" (Applied Biosystems, USA).

The results of the separation of the reaction products of sequencing were recorded by scanning with laser and the detection of four fluorescent dyes contained in all types of ddNTP.

1.7. Computer analysis of DNA sequences was performed using a personal computer using programs Chromas and BioEdit. The nucleotide sequences of the investigated DNA fragments were aligned with the designed, the identity of the synthesized fragments to the calculated ones was demonstrated. As a result, clones of *E. coli* cells were selected containing the full sequences of the target genes in the plasmids - the DNA sequences encoding the alpha defensin HNP-1 or HNP-2 or HNP-3.

**Example 2. Production of the plasmid DNA encoding alpha defensin HNP-1 or**

**HNP-2 or HNP-3, to conduct the study on laboratory animals.**

A colony of *E. coli* grown on LB-agar in a Petri dish with the addition of kanamycin (or ampicillin), was placed in 10 ml of a selective media. Cells grew over night at +37°C under constant stirring (250 rpm). The resulting cells were collected by centrifugation at 4000g. Further isolation and purification of the plasmid DNA was performed using EndoFree Plasmid Mega Kit (Qiagen), which allows to obtain non-pyrogenic DNA. Isolated plasmid DNA was analyzed with electrophoresis in a 0.8% agarose gel, its concentration was measured using fluorometry.

As a control solution water without addition of test preparation was used. 1,950 ml of water and 0.05 ml of test solution were added into a cell for measuring optical density with a volume of 2 ml, mixed, and the optical density was measured at a wavelength of 260 nm. Determination of the concentration of DNA was performed according to the formula:

$$C(\mu g/ml)=40A_{260}K,$$

where $A_{260}$-optical density of the preparation measured at a wavelength of 260 nm; K(μg/ml) - for DNA 50 μg/ml (50 μg/ml double-stranded DNA in water); 40 - dilution of the test preparation.

In the end it was determined that plasmid DNA pVAX1seq3 was obtained with a concentration of 3.4 mg/ml, pVR1012seq4 - 3.7 mg/ml, pVR1012seq5 - 4 mg/ml, pVAX1seq6 - 3,8 mg/ml, pVR1012seq9 - 4,1 mg/ml, pVAX1seq10 - 4 mg/ml, pVAX1seq11 - 4.3 mg/ml, pVR1012seq12 - 4.5 mg/ml, pcDNA3.1+seq11 - 4.4 mg/ml, pcDNA3.1+(var)seq12 - 4.3 mg/ml. The yield of plasmid DNA ranged from 3.4 mg to 4.5 mg from 1 1 of culture medium.

The purity of the obtained plasmid DNA preparation was judged by the ratio of optical density of the preparation measured at a wavelength of 260 nm to the optical density of the preparation measured at a wavelength of 280 nm ($A_{260}/A_{280}$) and ratio of optical density of the preparation measured at a wavelength of 260 nm to the optical density of the preparation measured at a wavelength of 230 nm ($A_{260}/A_{230}$). The measurements were performed in aqueous solution, as a reference solution water without addition of the test preparation was used.

For pure preparations of DNA $A_{260}/A_{280}$>1.80 and $A_{260}/A_{230}$>1.80 are characteristic. Defined in the experiment values corresponded to the values of the relations $A_{260}/A_{280}$ and $A_{260}/A_{230}$ for pure preparations, for all preparations of plasmid DNA received.

A quantitative determination of protein impurities was conducted in the obtained preparations of plasmid DNA pVAX1seq3, pVR1012seq4, pVR1012seq5, pVAX1seq6, pVR1012seq9, pVAX1seq10, pVAX1seq11, pVR1012seq12, pcDNA3.1+seq11, pcDNA3.1+(var)seq12 using the microBCA assay [Smith, P. K., et all, Measurement of protein using bicinchoninic acid. Analyt. Biochem. 150, 76-85 (1985)], measuring the optical density of the obtained colored protein complexes with copper and bicinchoninic acid at a wavelength of 562 nm. The sensitivity of the method microBCA assay is 0.5-20 μg/ml protein. The concentration of total protein in any of the studied preparations of the plasmid DNA did not exceed the norm (from 0.5 to 12 μg/mg of plasmid DNA).

The content of bacterial lipopolysaccharides was also determined in preparations of plasmid DNAs, using a gel-thrombus version of the LAL test, with a sensitivity of >0,25 EU/ml (ToxinSensor, GenScript, USA). A lysate of horseshoe crab *Limulus Polyphemus* amebocytes served as a LAL-reagent. LAL-reagent specifically reacts with bacterial endotoxins, the result of the enzymatic reaction there is a change in the reaction mixture proportional to the concentration of endotoxin. The results were evaluated according to the presence or absence of a dense thrombus at the bottom of the tube by turning the tube. The gel-thrombus was not formed during the study of a sample diluted 10 times, for preparations of plasmid DNAs pVAX1seq3, pVR1012seq4, pVR1012seq5, pcDNA3.1+(var)seq12 and 5 times for preparations of plasmid DNAs pVAX1seq6, pVR1012seq9, pVAX1seq10, pVAX1seq11, pVR1012seq12, pcDNA3.1+seq11, i.e. at the sensitivity of the method 2.5 EU/ml and 1.25 EU/ml, respectively, which, given the concentration of the plasmid DNA in the sample, confirms a valid indicator of purification from endotoxins.

**Example 3. Determination of the analgesic effect of plasmid DNA encoding alpha defensin HNP-1 or HNP-2 or HNP-3.**

3.1. Test "Hot plate"

A study of the analgesic activity of plasmid DNAs encoding alpha defensin HNP-1 or HNP-2 or HNP-3 was conducted, using the test "Hot plate" (hot plate).

The test "Hot plate" (Hot plate) was performed to measure the threshold of acute pain sensitivity and the potential analgesic effect of the studied preparations of plasmid DNA [Valdman A.V., Ignatov, Yu. D. Central mechanisms of pain. - L.: Nauka. - 1976]. The test is basical for the study of analgesic activity, it is used to identify the analgesic active compounds.

When placed on a hot surface, upon reaching the threshold of pain sensitivity by the animal, motor responses of anxiety are observed: a flick of the paws, licking of paws and jumping. In this test, the latency time since the placement of the animal on a hot surface to the first licking of the paws was considered. This method allows to determine the parameters: the analgesic activity of the tested object, the peak time of analgesia, duration of analgesia.

Mice of line BALB/C were used in the study, females, weighing 15 - 22 grams, of age 18 weeks. 17 groups of animals were formed in the study including control groups, each group consisted of 3 mice:

1 - negative control (saline was injected)
2 - pVAX1seq3
3 - pVR1012seq4
4 - pVR1012seq5
5 - pVAX1seq6
6 - pVR1012seq9
7 - pVAX1seq10
8 - pVAX1seq11
9 - pVR1012seq12
10 - pcDNA3.1+ seq11,
11 - pcDNA3.1+ (var)seq12
12 - analgin (50 mg/kg)
13 - morphine hydrochloride (10 mg/kg)
14 - pVAX1
15 - pVR1012
16 - pcDNA3.1+
17 - pcDNA3.1+(var)

5 mg/kg of the plasmid DNA were injected.

An appliance Hot plate-meter (Hotplate Analgesia Meter, Columbus Instruments, USA) was used.

Prior to the study test-systems were given 10 minutes to acclimate in the room for the study conduction. Animals were used only once, since the second putting of an animal on a thermostatic plate induced an immediate reaction to the touch of surface. The thermostat temperature was set 55°C. After the injection of the test substance, the animal was gently placed on the heating plate, and at the same time the "start" button on the control panel was pushed. Latency time of licking of the front and rear legs was noted (since the placement of the animal on the surface of the device before the first lick). After that, the "stop" button was pushed, and the animal was removed from the hot surface. Other behavioral responses were ignored. To reduce the likelihood of heat damage of the pads, the maximum time of the experiment did not exceed 60 sec. To determine the peak time of the analgesic action of the preparation, latency time of licking of the front and hind paws was measured in the control group (saline) (point 0) and after 2 h, 12 h, 24 h after preparation administration in the tested groups. The surface of the appliance was wiped with a cloth moistened with disinfectant (0.5% chlorhexidine-digluconate in 70% ethanol), before putting on it the next animal [Methodical recommendations for the students. The pharmaceutical faculty of GOU VPO MMA im. I. M. Sechenov of the Russian Ministry Of Health. - Moscow. - 2006].

The analysis of data of determination of the analgesic activity of the preparation was performed. For plasmid DNA which has shown the best result, ED50 was determined, i.e. the dose needed for the demonstration of 50% analgesic activity of the preparation.

The results of the experiment are shown on Fig.1. Preparations of all analyzed plasmid DNAs encoding the defensin HNP-1 or HNP-2 or HNP-3 exhibit a significant analgesic action, not inferior in effectiveness to morphine and exceeding the analgesic activity of analgin. A small degree of analgesia was observed in 2 h after injection of plasmids, then analgesia got strengthened, and in 12 and 24 h the same high level of analgesia was observed, indicating the maintenance of the effect.

In experiment with application of various doses of plasmid DNA which has shown the best result in the previous experiment (pVAX1seq3), a dose-dependent nature of analgesia in the range from 1 to 10 mg/kg was demonstrated (Fig.2). Wherein after 12 hours the indicators when using plasmid DNA pVAX1seq3 in minimal concentration of 1 mg/kg were almost identical to those in the group of morphine hydrochloride 10 mg/kg use. After 24 h the indicator in the group of plasmid DNA in a minimal concentration exceeded that of the group of use of morphine hydrochloride 10 mg/kg, almost in 2 times. The effect of analgin was observed only after 2 h after administration of analgesic, the response after 12 h and 24 h was similar to that in the control group. When using the plasmid DNA pVAX1seq3 at a concentration of 5 mg/kg and 10 mg/kg, a significant increase (up to 15%) of a latent period was observed, compared to the group with a minimum concentration of plasmid DNA, wherein the difference between these two groups was small.

3.2. Study of the concentration of beta-endorphin in the serum of mice.

Since the change in the concentration of beta-endorphin in blood plasma can serve a criterion for assessing the effectiveness of anesthesia [Beta-endorphin is a marker of the effectiveness of analgesia for acute pain and chronic pain syndrome in cancer patients / Z. V. Pavlova [and others] // Problems of clinical medicine. - 2007 . - N1 . - S. 36-40 . - ISSN 1817-8359], a study of concentration of beta-endorphin in the serum of mice after injection of plasmid DNA was conducted, while introduction of the plasmid DNA encoding HNP-2 or HNP-3 (pVR1012seq4) as example, using the kit ELISA Kit for Beta-Endorphin (bEP) Mus musculus (Mouse) CEA806Mu (Life science Inc.).

The results of the study are shown in Fig.3. It is seen that the level of beta-endorphin increased significantly with the introduction of the plasmid DNA pVR1012seq4, despite the fact that after introduction of that plasmid DNA without insert of the target gene and in the control group a significant increase in the level of beta-endorphin was not observed throughout the whole experiment.

Thus, the possibility of creating different variants of the plasmid DNA encoding HNP-1 or HNP-2 or HNP-3, their obtaining with the use of a bacterial producer, and an analgesic effect of such plasmid DNA are demonstrated, in any of the variants that match the specified criteria, even in a minimal concentration of 1 mg/kg (10 times less than of morphine hydrochloride), and also the safety of using such plasmid DNA is demonstrated: the animals survived, side effects were not observed.

## Claims

**1.** A plasmid DNA for transient expression in mammalian cells, represented by a skeleton that contains prokaryotic elements, an origin of replication and a marker gene, and eukaryotic elements, a strong promoter, a leader sequence of mRNA, and also regulatory sequences for the mentioned elements, at least one site for cloning of a gene of interest and at least one site for annealing of at least one primer for analysis of the composition of the plasmid DNA, and a polynucleotide represented by a secretory sequence, a fragment encoding human alpha defensin HNP-1 or

HNP-2 or HNP-3, codon-optimized for expression in mammalian cells, and a termination sequence.

2. Plasmid DNA according to claim 1, **characterized by** the fact that preproprotein is represented by SEQ ID NO:1 or SEQ ID NO:2 or SEQ ID NO:7 or SEQ ID NO:8.

3. Plasmid DNA according to claim 1 or claim 2, **characterized in that** the polynucleotide is represented by SEQ ID NO:3 or SEQ ID NO:4 or SEQ ID NO:5 or SEQ ID NO:6 or SEQ ID NO:9 or SEQ ID NO: 10 or SEQ ID NO:11 or SEQ ID NO:12.

4. A bacterial cell producing the plasmid DNA according to claim 1 or claim 2 or claim 3.

5. An analgesic agent based on the plasmid DNA according to claim 1 or claim 2 or claim 3 in an effective amount, also containing a pharmaceutically acceptable excipient, for use as a medicament in a method of treating pain in mammals

## Patentansprüche

1. Plasmid-DNA für die transiente Expression in Säugetierzellen, dargestellt durch ein Gerüst, das prokaryotische Elemente, einen Replikationsursprung und ein Markergen sowie eukaryotische Elemente, einen starken Promotor, eine Leader-Sequenz von mRNA, sowie regulatorische Sequenzen für die genannten Elemente, mindestens eine Stelle zur Klonierung eines interessierenden Gens und mindestens eine Stelle zum Anlagern von mindestens einem Primer zur Analyse der Zusammensetzung der Plasmid-DNA, enthält, und durch ein Polynukleotid, dargestellt durch eine sekretorische Sequenz, ein Fragment, das menschliches Alpha-Defensin HNP-1 oder HNP-2 oder HNP-3, das für die Expression in Säugetierzellen codonoptimiert ist, kodiert, und durch eine Terminationssequenz.

2. Plasmid-DNA nach Anspruch 1, **dadurch gekennzeichnet, dass** Präproprotein durch SEQ ID NO:1 oder SEQ ID NO:2 oder SEQ ID NO:7 oder SEQ ID NO:8 dargestellt wird.

3. Plasmid-DNA nach Anspruch 1 oder Anspruch 2, **dadurch gekennzeichnet, dass** das Polynukleotid durch SEQ ID NO:3 oder SEQ ID NO:4 oder SEQ ID NO:5 oder SEQ ID NO:6 oder SEQ ID NO:9 oder SEQ ID NO:10 oder SEQ ID NO:11 oder SEQ ID NO:12 dargestellt wird.

4. Eine Bakterienzelle, die die Plasmid-DNA nach Anspruch 1 oder Anspruch 2 oder Anspruch 3 produziert.

5. Ein Analgetikum auf Basis der Plasmid-DNA nach Anspruch 1 oder Anspruch 2 oder Anspruch 3 in einer wirksamen Menge, das auch einen pharmazeutisch annehmbaren Hilfsstoff enthält, zur Verwendung als Medikament in einem Verfahren zur Schmerzbehandlung bei Säugetieren.

## Revendications

1. Un ADN plasmidique pour l’expression transitoire dans des cellules de mammifère, représenté par un squelette, contenant des éléments procaryotes, une origine de réplication et un gène marqueur, et des éléments eucaryotes, un promoteur fort, une séquence leader de l’ARNm, et aussi des séquences régulatrices des éléments mentionnés, au moins un site de clonage d’un gène d’interêt et au moins un site d’annelage d’au moins une amorce pour l’analyse de la composition de l’ADN plasmidique, et un polynucléotide représenté par une séquence de sécrétion, un fragment codant pour l’alpha défensine humaine PNH-1 ou PNH-2 ou PNH-3 optimisés en codons pour l’expression dans des cellules de mammifère et une séquence de terminaison.

2. L’ADN plasmidique selon la revendication 1, **caractérisé en ce que** la préproprotéine est représentée par SEQ ID NO:1, ou SEQ ID NO:2, ou SEQ ID NO:7, ou SEQ ID NO:8.

3. L’ADN plasmidique selon la revendication 1 ou 2, **caractérisé en ce que** le polynucléotide est représenté par SEQ ID NO:3, ou SEQ ID NO:4, ou SEQ ID NO:5, ou SEQ ID NO:6, ou SEQ ID NO:9, ou SEQ ID NO: 10, ou SEQ ID NO: 11, ou SEQ ID NO: 12.

4. Une cellule bactérienne produisant l’AND plasmidique selon la revendication 1, ou 2, ou 3.

**5.** Un agent analgésique basé sur l'ADN plasmidique selon la revendication 1, ou 2, ou 3 en une quantité efficace, aussi contenant un excipient pharmaceutiquement acceptable, pour l'utilisation d'un médicament dans un procédé de traitement de la douleur chez mammifère.

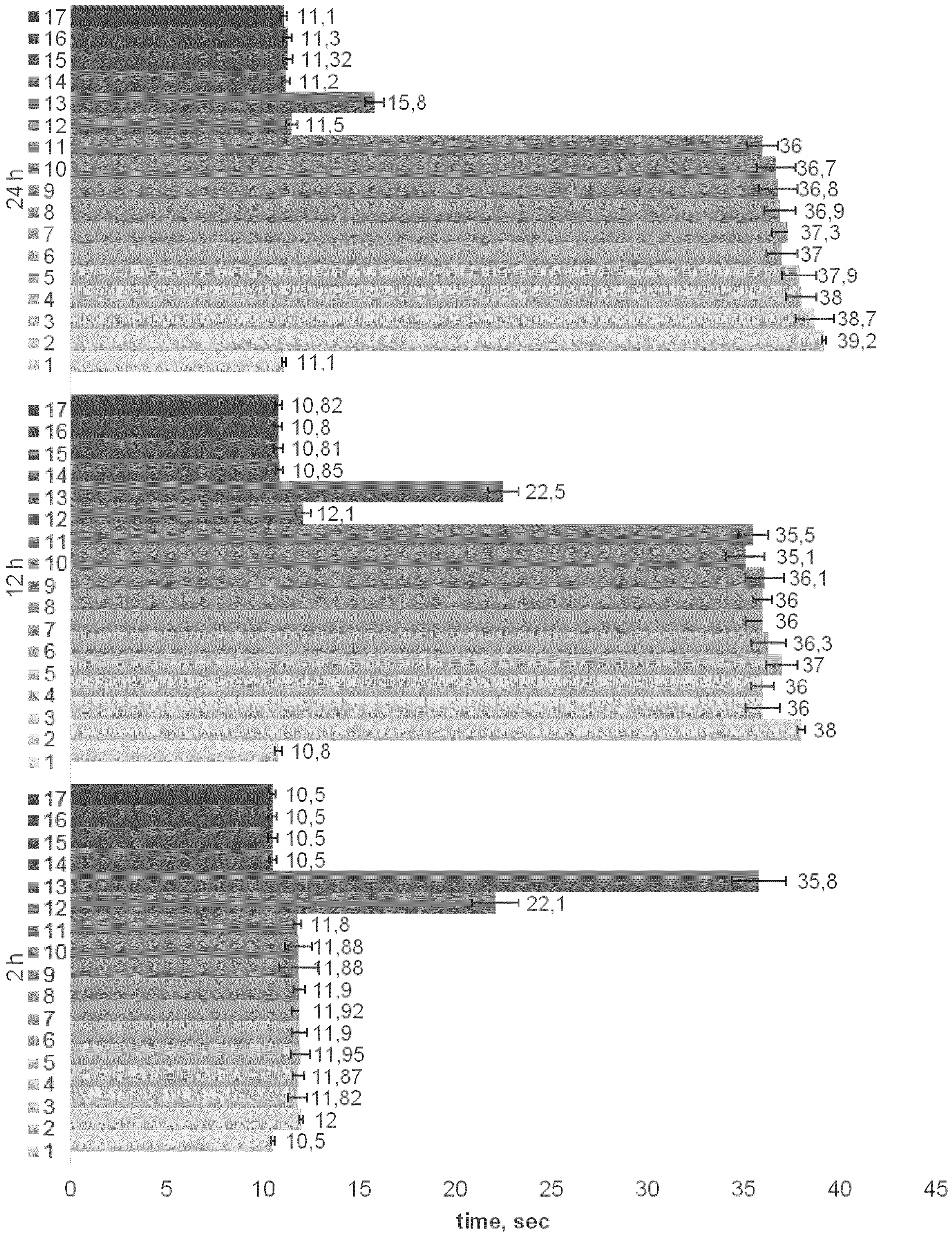
24 h
17 11,1
16 11,3
15 11,32
14 11,2
13 15,8
12 11,5
11 36
10 36,7
9 36,8
8 36,9
7 37,3
6 37
5 37,9
4 38
3 38,7
2 39,2
1 11,1
12 h
17 10,82
16 10,8
15 10,81
14 10,85
13 22,5
12 12,1
11 35,5
10 35,1
9 36,1
8 36
7 36
6 36,3
5 37
4 36
3 36
2 38
1 10,8
2 h
17 10,5
16 10,5
15 10,5
14 10,5
13 35,8
12 22,1
11 11,8
10 11,88
9 11,88
8 11,9
7 11,92
6 11,9
5 11,95
4 11,87
3 11,82
2 12
1 10,5
0 5 10 15 20 25 30 35 40 45
time, sec

Fig. 1

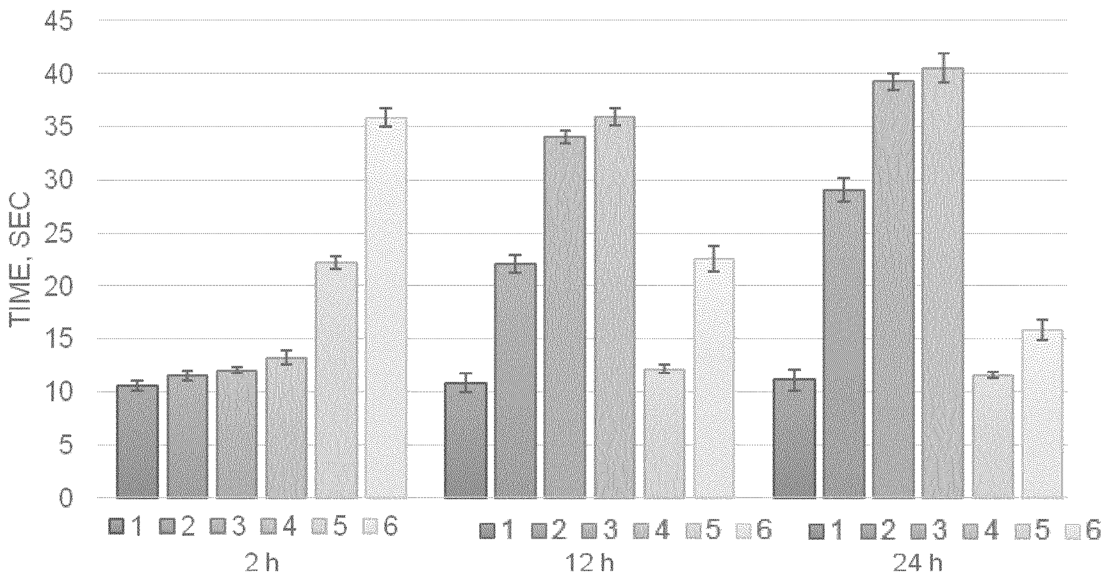
45
40
35
30
25
20
15
10
5
0
TIME, SEC
1 2 3 4 5 6
2 h
1 2 3 4 5 6
12 h
1 2 3 4 5 6
24 h

Fig.2

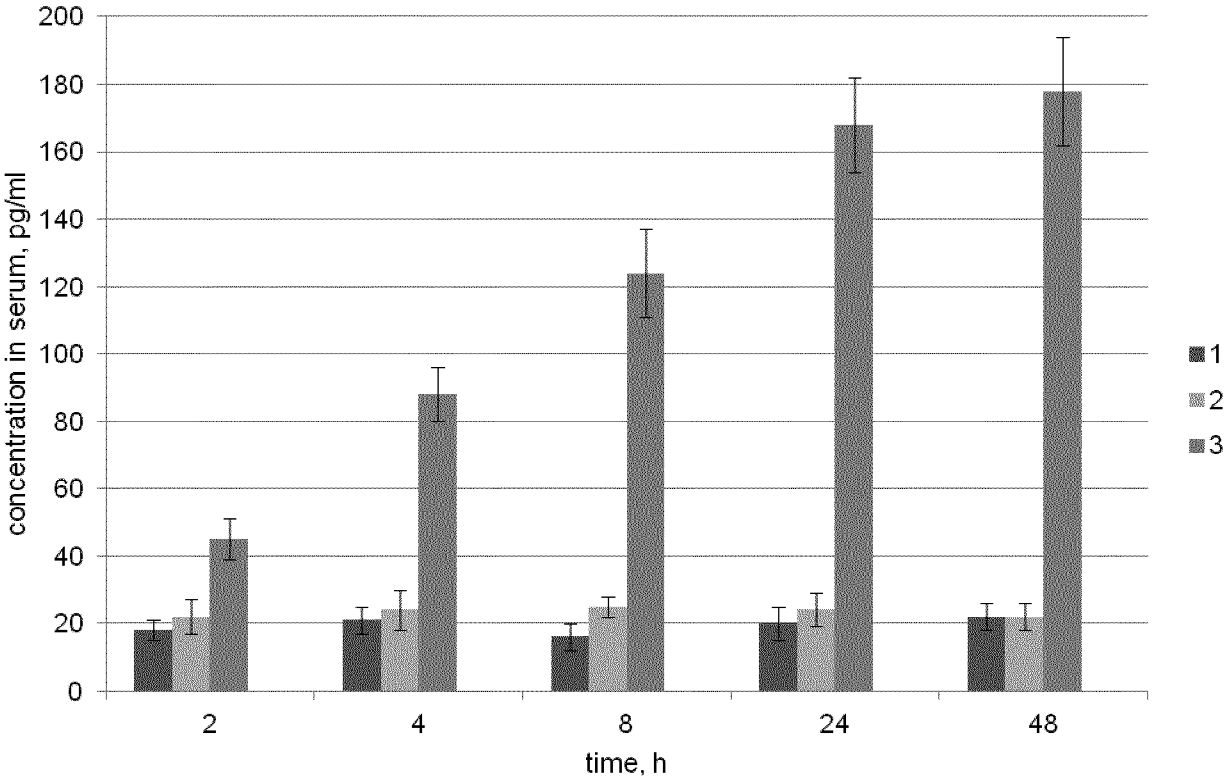
200
180
160
140
120
100
80
60
40
20
0
concentration in serum, pg/ml
2
4
8
24
48
time, h
1
2
3

Fig.3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4459225 A
- EP 0952159 B1
- RU 2500685 C2
- WO 2008020778 A1
- US 2008009448 A1
- SK 278170 B6
- CN 102477436 A
- RU 2368621 C1
- RU 2404245 C1
- RU 2415866 C1
- RU 2498995 C2
- CN 1487085 A
- CN 102876683 A
- EP 0625162 B1
- WO 2014023129 A1
- CN 102286079 B
- CN 103374066 A
- CN 102628048 A
- EP 1336409 B1
- US 7592309 B2
- CN 101591668 A
- CN 102977201 A
- US 5776896 A
- US 6670329 B2
- US 6613745 B1
- US 7902152 B2
- US 7294697 B2
- CN 1345751 A
- WO 0212310 A1
- WO 0204504 A1
- CN 1352195 A
- US 2001016336 A1
- EP 0837128 A2
- US 5912335 A
- US 5955309 A
- US 2003162945 A1
- WO 0200725 A2
- AU 779993 B2
- US 6998255 B1
- US 5874252 A
- RU 2389509 C2
- RU 2429013
- RU 2338555 C2
- RU 2406728 C2
- RU 2427387 C2
- RU 2011149263 A
- RU 2473564 C2
- RU 2011105280 A
- WO 2012009075 A1
- US 7737250 B2
- US 2013296247 A1
- RU 2440130 C2
- WO 2009064497 A1
- RU 2011110169 A
- RU 2007119313 A
- RU 2266129 C2
- US 8466267 B2
- US 2003108906 A
- US 2003134301 A1
- US 2003138803 A1
- US 2004058326 A1
- RU 98118091 A
- CA 2219437 A1
- US 6566327 B1
- WO 2013070076 A1
- US 7261882 B2
- RU 2472807 C2
- RU 2012124438 A
- RU 2008103312 A
- RU 2011140335 A
- RU 2011104228 A
- RU 2515108 C2
- RU 2011104348 A
- RU 2004114989 A
- RU 2433133 C2
- RU 2012154210 A
- RU 2415151 C2
- RU 2467765 C2
- RU 2483747 C2
- RU 2434637 C2
- RU 2345785 C2
- US 2004053244 A, A1
- US 2003073115 A, A1
- WO 0168843 A, A1
- US 6696408 B, B1
- US 6525021 B, B1
- US 7939493 B, B2
- RU 2012106468 A
- RU 2011136454 A
- RU 2430750 C2
- RU 2180852 C2
- JP 2000198744 A
- RU 2385878 C2
- RU 2012106820 A
- WO 9104041 A1
- US 5446026 A
- US 5710024 A
- WO 9803534 A1

- US 7193070 B2
- US 6033872 A
- WO 9928492 A1
- US 5824504 A
- US 6277960 B1
- US 6277977 B1
- US 5955308 A
- US 6221627 B1
- US 2002106766 A1
- US 5994098 A
- US 6174994 B1
- US 6037146 A
- US 5874243 A
- WO 0166716 A1
- EP 0870825 B1
- US 5972606 A
- CN 1978466 B
- WO 9942480 A
- US 6008050 A
- WO 2010063122 A1
- US 4649191 A
- KR 20090041066 A
- RU 2010144778 A
- RU 2508296 C2
- RU 2422459 C1
- RU 2298559 C2
- RU 2398776 C2
- RU 2011149796 A
- US 8188048 B2
- EP 0096592 B1
- AU 2002228260 B2
- WO 2009043461 A1
- US 8372817 B2
- US 8575330 B2
- US 8361985 B2
- WO 2013126963 A1
- US 7166279 B2
- WO 2013177484 A1
- EP 1425403 B1
- CN 101518655 A
- JP 4803789 B
- WO 2013034157 A1
- CN 101648011 A
- US 2006154886 A1
- RU 2014145169


**Non-patent literature cited in the description**


- **J. A. WOLFF et al.** *Science,* 1990, vol. 247, 1465
- **G. ASCADI et al.** *Nature,* 1991, vol. 352, 815
- **H. LIN et al.** *Circulation,* 1990, vol. 82, 2217
- **R. N. KITSIS et al.** *Proc. Natl Acad. Sci.,* 1991, vol. 88, 4138
- **E. HANSEN.** *FEBS Lett.,* 1991, vol. 290, 73
- **S. JIAO et al.** *Hum. Gene Therapy,* 1992, vol. 3, 21
- **J. A. WOLFF et al.** *Human Mol. Genet.,* 1992, vol. 1, 363
- **FURTH et al.** *Analytical Biochemistry,* 1992, vol. 205, 365-368
- **MECKERT, P. C ; HONTEBEYRIE-JOSKOWICZ, M. ; CHAMBO, J. ; LEVIN, M. ; LAGUENS, R. P.** Trypanosoma cruzi: Aberrant expression of class II major histocompatibility complex molecules in skeletal and heart muscle cells of chronically infected mice. *Exp. Parasitol.,* 1991, vol. 72, 8-14
- **HOHLFELD ; ENGEL, A. G.** The immunobiology of muscle. *Immunol. Today,* 1984, vol. 15, 269-274
- **MANTEGAZZA, R. ; BERNASCONI, P.** Cellular aspects of myositis. *Curr. Opin. Rheumatol.,* 1994, vol. 6, 568-574
- **HARTIKKA J ; SAWDEY M ; CORNEFERT-JENSEN F ; MARGALITH M ; BARNHART K ; NOLASCO M ; VAHLSING HL ; MEEK J ; MARQUET M ; HOBART P.** An improved plasmid DNA expression vector for direct injection into skeletal muscle. *Hum Gene Ther.,* 20 June 1996, vol. 7 (10), 1205-17
- **SAADE F ; PETROVSKY N.** Technologies for enhanced efficacy of DNA vaccines. *Expert Rev Vaccines,* February 2012, vol. 11 (2), 189-209
- **PARKER SE ; BORELLINI F ; WENK ML et al.** Plasmid DNA malaria vaccine: tissue distribution and safety studies in mice and rabbits. *Hum. Gene Ther.,* 1999, vol. 10 (5), 741-758
- **MANTHORPE M ; CORNEFERT-JENSEN F ; HARTIKKA J et al.** Gene therapy by intramuscular injection of plasmid DNA: studies on firefly luciferase gene expression in mice. *Hum. Gene Ther.,* 1993, vol. 4 (4), 419-431
- **WILLIAMS JA ; CARNES AE ; HODGSON CP.** Plasmid DNA vaccine vector design: impact on efficacy, safety and upstream production. *Biotechnol. Adv.,* 2009, vol. 27 (4), 353-370
- **CAZEAUX N ; BENNASSER Y ; VIDAL PL ; LI Z ; PAULIN D ; BAHRAOUI E.** Comparative study of immune responses induced after immunization with plasmids encoding the HIV-1 Nef protein under the control of the CMV-IE or the muscle-specific desmin promoter. *Vaccine,* 2002, vol. 20 (27-28), 3322-3331

- **KOZAK M.** Recognition of AUG and alternative initiator codons is augmented by G in position +4 but is not generally affected by the nucleotides in positions +5 and +6. *EMBO J.,* 1997, vol. 16 (9), 2482-2492
- **FRELIN L ; AHLEN G ; ALHEIM M et al.** Codon optimization and mRNA amplification effectively enhances the immunogenicity of the hepatitis C virus nonstructural 3/4A gene. *Gene Ther.,* 2004, vol. 11 (6), 522-533
- **MONTGOMERY DL ; SHIVER JW ; LEANDER KR et al.** Heterologous and homologous protection against influenza A by DNA vaccination: optimization of DNA vectors. *DNA Cell Biol.,* 1993, vol. 12 (9), 777-783
- **NORMAN JA ; HOBART P ; MANTHORPE M ; FELGNER P ; WHEELER C.** Development of improved vectors for DNA-based immunization and other gene therapy applications. *Vaccine,* 1997, vol. 15 (8), 801-803
- **AZZONI AR ; RIBEIRO SC ; MONTEIRO GA ; PRAZERES DMF.** The impact of polyadenylation signals on plasmid nucleases-resistance and transgene expression. *J Gene Med.,* 2007, vol. 9, 392-402
- **SAADE F ; PETROVSKY N.** Technologies for enhanced efficacy of DNA vaccines. *Expert Rev Vaccines,* February 2012, vol. 11 (2), 189-209
- **SHEETS RL ; STEIN J ; MANETZ TS ; DUFFY C ; NASON M ; ANDREWS C ; KONG WP ; NABEL GJ ; GOMEZ PL.** Biodistribution of DNA plasmid vaccines against HIV-1, Ebola, Severe Acute Respiratory Syndrome, or West Nile virus is similar, without integration, despite differing plasmid backbones or gene inserts. *Toxicol Sci.,* June 2006, vol. 91 (2), 610-9
- **SHEETS RL ; STEIN J ; MANETZ TS ; ANDREWS C ; BAILER R ; RATHMANN J ; GOMEZ PL.** Toxicological safety evaluation of DNA plasmid vaccines against HIV-1, Ebola, Severe Acute Respiratory Syndrome, or West Nile virus is similar despite differing plasmid backbones or gene-inserts. *Toxicol Sci.,* June 2006, vol. 91 (2), 620-30
- **GANZ, T.** Immunology: Versatile defensins. *Science,* 2002, vol. 298, 977-979
- **LEHRER, R. I. ; GANZ, T.** Defensins of vertebrate animals. *Curr. Opin. Immunol.,* 2002, vol. 14, 96-102
- **O. LEVY.** Antimicrobial proteins and peptides: anti-infective molecules of mammalian leukocytes. *J. of Leukocyte Biology,* 2004, vol. 76, 909-926
- **A. S. BUDIKHINA ; B. V. PINEGIN.** Defensins - multifunctional cationic peptides of the person. *Immunopathology, Allergology, Infectology,* 2008, vol. 2, 31-40
- **ZV PAVLOVA.** *Problems of Clinical Medicine,* 2007, (1), ISSN 1817-8359, 36-40
- **EDDY N.B. ; LEIMBACH D. J.** *Pharmacol Exp Ther.,* 1953, vol. 107 (3), 385-93
- **HARTIKKA J ; SAWDEY M ; CORNEFERT-JENSEN F ; MARGALITH M ; BARNHART K ; NOLASCO M ; VAHLSING HL ; MEEK J ; MARQUET M ; HOBART P.** An improved plasmid DNA expression vector for direct injection into skeletal muscle. *Hum Gene Ther.,* 20 June 1996, vol. 7 (10), 1205-17
- Cloning Vectors. Elsevier, 1985
- **WILLIAMS JA ; CARNES AE ; HODGSON CP.** Plasmid DNA vaccine vector design: impact on efficacy, safety and upstream production. *Biotechnol Adv.,* July 2009, vol. 27 (4), 353-70
- **SMITH, P. K.** Measurement of protein using bicinchoninic acid. *Analyt. Biochem.,* 1985, vol. 150, 76-85
- **VALDMAN A.V. ; IGNATOV, YU. D.** *Central mechanisms of pain,* 1976
- Methodical recommendations for the students. **I. M. SECHENOV.** The pharmaceutical faculty of GOU VPO MMA im. Russian Ministry Of Health, 2006
- **Z. V. PAVLOVA.** Beta-endorphin is a marker of the effectiveness of analgesia for acute pain and chronic pain syndrome in cancer patients. *Problems of clinical medicine,* 2007, (1), ISSN 1817-8359, 36-40